# FASCICULE DE BREVET EUROPEEN

(11) **EP 1 831 391 B1**
(45) Date de publication et mention de la délivrance du brevet: **31.12.2008**
(21) Numéro de dépôt: 05825881.5
(22) Date de dépôt: 20.12.2005
(51) Int. Cl.: C12Q 1/18

(54) **UTILISATION D'INHIBITEURS DE LA METHIONINE SYNTHASE POUR LE TRAITEMENT DES MALADIES FONGIQUES DES CULTURES**
VERWENDUNG VON METHIONINSYNTHASE-INHIBITOREN ZUR BEHANDLUNG VON PILZKRANKHEITEN BEI NUTZPFLANZEN
USE OF METHIONINE SYNTHASE INHIBITORS FOR THE TREATMENT OF FUNGAL DISEASES OF CROPS

(30) Priorité: 21.12.2004 FR 0413628
(43) Date de publication de la demande: 12.09.2007
(73) Titulaire: BAYER CROPSCIENCE SA, 69009 Lyon Cédex 09 (FR)
(72) Inventeur: DROUX, Michel, F-69160 Tassin La Demi Lune (FR); LEBRUN, Marc-Henri, F-69002 Lyon (FR)
(74) Mandataire: Monconduit, Hervé
(86) Numéro de dépôt international: PCT/EP2005/014209
(87) Numéro de publication internationale: WO 2006/066974

(56) Documents cités:
- US-A1- 2004 191 849
- PASCON RENATA C ET AL: "Cryptococcus neoformans methionine synthase: expression analysis and requirement for virulence" MICROBIOLOGY (READING), vol. 150, no. Part 9, septembre 2004 (2004-09), pages 3013-3023, XP002346120 ISSN: 1350-0872
- DRUMMOND J T ET AL: "Characterization of Nonradioactive Assays for Cobalamin-Dependent and Cobalamin-Independent Methionine Synthase Enzymes" ANALYTICAL BIOCHEMISTRY, ACADEMIC PRESS, SAN DIEGO, CA, US, vol. 228, no. 2, 1995, pages 323-329, XP002297999 ISSN: 0003-2697
- HUANG LONGQUAN ET AL: "Assays of methylenetetrahydrofolate reductase and methionine synthase activities by monitoring 5-methyltetrahydrofolate and tetrahydrofolate using high-performance liquid chromatography with fluorescence detection" ANALYTICAL BIOCHEMISTRY, vol. 299, no. 2, 15 décembre 2001 (2001-12-15), pages 253-259, XP002346121 ISSN: 0003-2697
- SOLOMON PETER S ET AL: "Methionine synthase, a gene required for methionine synthesis, is expressed in planta by Cladosporium fulvum" MOLECULAR PLANT PATHOLOGY, vol. 1, no. 5, septembre 2000 (2000-09), pages 315-323, XP002346122 ISSN: 1464-6722

### SEQUENCE LISTING

<110> Bayer CropScience S.A.
<120> utilisation d'inhibiteurs de la méthionine synthase pour le traitement des maladies fongiques des cultures
<130> BCS04-4024
<160> 20
<170> PatentIn version 3.3
<210> 1
   <211> 6500
   <212> DNA
   <213> Magnaporthe grisea
<220>
   <221> gene
   <222> (1)..(6500)
<400> 1
   ggaagaaatc actgcgaaca acacacatct tggcccctga agctaggcca gtcttgtcaa 60
   gcagtggcaa cgatggtaaa agcgagcgtt ggaaggattt ggagctcgtc attcccaaga 120
   gacaggatga caacctctat caggtatgtt tcatatctcc ctgacttttg aattatccgg 180
   cctgggtata gcccctattg ctaacctgcg tctgttactt tcacagaacc tcgaggatga 240
   ggagttgctc aagatatcgc agcagatagg gagtcatatg gatagtatgc aaggaaatct 300
   acggcaggtt gatggaattc ttccagcgat ggaaataaca aaggccgcgc ttcacgatgt 360
   gttattgaaa cactccacca agtgtgagga ggtactgctt ggataagcac gcctatacta 420
   ggttgggcaa aaggaccaaa gaaggttcgt ttgatacttg agatgggggc agtaatgtta 480
   tcgtatgaat aatgagtcac catgtaaacg attgacagtc tgcaatcgga ggctaacgtc 540
   ttcggcttcg gcttgttgga aaatcagacg agttcggact ggcgagtggg ttctcacgag 600
   attgaggcca atgcttgccc gaacagtcaa ccaatttact acttccagcg gtgctactta 660
   ataatcgatg cgcgtttggg cctagcacga cgtcggggtg tttgttccat gtaaaaggca 720
   agtgagctta tgttgtagtg tagggaagaa ctttgagctt accatccata caaaacaagg 780
   caatacacgg gaggtttaaa attgccctgc aaacttttga cctccggtaa actccaggtc 840
   aatttaaacc tcgattgtag cgcaagattc gacatctctg tcgaacaagc tctgatttgt 900
   cgggggtcca tgccggttgc catggcgccc gacccggcct ccgatcaacg gacagttcga 960
   accacattgg atgatatcta ggctggactg cgtggtatac gagaccataa ctaatcagga 1020
   atgaagcccc agccaggtac agtacatatt gcaacctaaa gcacacaagc tatggtaagt 1080
   agtgggcata ccgggtccag ccgacgggtg agttttgctg ggaccctcct tgtcattttt 1140
   attgcagtaa gatataggtg ggcatacaaa gtaaaagcgt gatttacggt acggtacgga 1200
   gcaccctcgg tactctggtt tagttacctg caatgcgacg tgtcaccccg ttcgcgacgt 1260
   ctaggcaagg tagcggtgtg atattgtaca gtgtgcttga tagttattgt aggagttgga 1320
   cccacgtcaa aattcctctt cggtagcatt cgcaccaagc gattggcaga aaatgacggg 1380
   attgggattg aaatttctct gcaaaacctt aaaccttgct gagcccctcc ctaatgttgc 1440
   cccgcgctgt ttaagaggtc gaagcttcga actagagctc tctgcgcccc accgttgtgc 1500
   ccctccacga gtcgccctac gctttcatca cactttcgac aaccgctcat cggagcagca 1560
   ttggactctt gctccttttg cgaagcaatt cagcaccggg agaggcagcg cagtcagtca 1620
   gctgaggtgt ttacgagctt gttggtacct ttcccaaagg agacaggtac cgaagcttgc 1680
   aaagatacct cagacgcttg agatttcagt cgaaaccagg gtctgggccc cacaaaattt 1740
   tgccgttttt cccctccttc tttccatatg aagtctgtcc tcccctccat cccgttcctc 1800
   tttaccaaca gatttttcca ttccctttct catacttttc ttctgtctgc atacaagctg 1860
   gcacaaggtg aggctctggg agaaaatagt gagtgacttg agctttcacc ctctccgggt 1920
   tgtcgatgtc gaattgctag tctaacttct gcgattccca tttattccca gtcaccgaag 1980
   ccgtccaact cataacaaca ctcacattga gctcctaaaa tcgccggcga aaattcacac 2040
   ttgagcaaaa tggttcaatc agcgattctc ggtttccccc gcatgggtgt gaaccgtgac 2100
   ctgaagaagg ccactgaagc ttgtatgtct tgtcatcact gcaaaccacc aaggtctggc 2160
   tccggatgat cgctgctaac accaattatc tgttagactg ggccggcaag atctcgcagg 2220
   acgacctcct tgccgaggct aagaggctca gactcgccca ctggaagatc cagaaggatg 2280
   ccggtgtcga catcatcccc agcaatgatt ttgctttgta cgaccaggtc ctttcacaca 2340
   tccaggactt cggtgtgagt accatgccat cgagagcttt agtactttag ggatagccag 2400
   ctgacacatt ggtaatgtat taggccgttc ccgaaaggta ctcaagctcc aagctgaacc 2460
   ccgtcgacga gtacttcgcc atgggtcgtg gtcaccagaa ggatggtgtc gatgttccca 2520
   gcttggagat ggtcaagtgg ttcgactcaa actaccacta cgtcaagccc actctccagg 2580
   acaaccagac cttcacgctt acggccaacc ccaaggctgt gaatgagttc aacgaggcca 2640
   aggaggctgg catcaacacc cgccccgtcc tcgttggtcc cgtttctttc cttcacctcg 2700
   ccaaggctga ccgtggtcag tctgttgacc ccatcgacct ccttgacaag cttgtccccg 2760
   tttacgagga gctcctcgcc aagctcaagg ccgctggtgc cgagactgtc cagattgacg 2820
   agcctgtcct cgtcttcgac cttcccgcca aggtcaaggc tgctttcaag cccacatatg 2880
   agaagtttgc cagcctgggt gacaagatcc ccaagctcgt tttcgccaca tacttcggtg 2940
   acatcgtcca caatcttgac ctcgtcccca aggacgtcta cgccgtccac gtcgacctcg 3000
   tcaggaaccc tgagcagttg gaaactgttg ttggtgccct gggccccaag accattcttt 3060
   ctgctggtat cgtcgatggc cgtaacatct ggaagaccaa cttccagaag gccattgaga 3120
   ctgttgagag tgcgatccag aagctcggca aggagcgtgt cattgttgcc acttccagct 3180
   ctctccttca cactccccac acactagcga gcgagaagaa gcttgaccct gaaatcgccg 3240
   actggttctc atttgcctct gagaaggccg tcgaggttgc catcatcgcc aaggccgtca 3300
   ctgagggccc tgctgctgtc cgcgagcagc tcgaggccaa cgccaagtcg atgaacgctc 3360
   gcgccacctc gagcagaaca aatgacccca aggtcaagga gaggcagtca aagattgtcg 3420
   agtcagacta caaccgcaag tcggagttcc ctacccgtat ttcgcagcag caggccaagc 3480
   ttaaccttcc tctctttccc actactacca tcgggtcctt cccccagacc cagactatcc 3540
   gcgcccagcg tgccaagctc accaagaagg aaattgacgc tgagcaatac gccaagttca 3600
   tcgaggagga gattgagaac aatgtaaaga tccaggaaga gctcggtctg gatgtcttcg 3660
   tccacggtga gcccgagcgt aacgacatgg tgcaattctt tggtgagcgc ctggacggtt 3720
   atgccttcac cacgcacgcc tgggttcaga gctacggttc ccgctgcgtc cgtcctccca 3780
   tcattgtcgg tgacatctct cgcccggcac cgatgactgt caaggaatca aggtacgctg 3840
   tcgagatttc caagaagccc atgaagggta tgttgacggg ccccgtcacc tgcctgaggt 3900
   ggtcgttccc ccgtgacgat gtgcaccagt ccgtccaagc tgagcagctc gctcttgctc 3960
   tccgtgacga ggttgttgac cttgagaagg ctggtgtcga cgtcatccag gtcgacgagc 4020
   ctgctctccg tgagggtctg cccctccgct ctggtaagga gcgcgatgct tacctccagt 4080
   gggctgtcaa ggctttcaag ctctcgacct gtggtgtcga ggactcgact cagatccact 4140
   cgcacttctg ctactctgag ttccaggact tcttccacgc cattgctgcc cttgatgccg 4200
   acgttctgtc catcgagaac agcaagtctg atgccaagct gctgaaggtg ttcgtcgact 4260
   cggcttaccc ccgccacatc ggccccggtg tctacgacat ccactccccc cgtgttccca 4320
   gcgaacagga gatcaaggac cgcatcgagg agatgcttca gtacctcaag cctgagcagc 4380
   tctggatcga ccctgactgc ggtctgaaga cccgccagtg gaaggagacc aaggaggctc 4440
   tcaccaacat ggtcaacgcc gccaagtact tccgtgccaa gtacgccaaa taagctgcaa 4500
   gtcggctttt ttctttctct aatgttttac tcatctggtt tttcggcgtt tttgagccca 4560
   cgcgtttccg tggcatgact tgcgggatct ggtcttcgat ttcaacatcg gcgttttttt 4620
   tttcttgatt ctgggatatg atatcaaaag tgcaagcgat aagtctccga aatacaggtg 4680
   ttcgggtggg tttaaaaact ggtggttggg ttcatgggaa cggcgtgagg atcattcaac 4740
   atttggcaag gaataccaaa agggttccgg agacctagag ggaattttgt ccatcaaccg 4800
   gacttcccga atcactcata cccctattct atttcctttt tccctttttt tttgtttttg 4860
   gtacgaagtc ctttttcatc ttcttgcaac ataccatcag atataatgac gggagtattg 4920
   gggcagagaa gtaggaaggg ccactgatgt cagtacagac tgaccgtggc agttagatag 4980
   tcacactata ggaatgaatg agacccaatt caactgggta atagactgtc ctggacttgg 5040
   ctttttttcc ttggggcatt attatcaagc agccactttc agtagtaaac aaatactctg 5100
   gggttccacc actataatac agatggagat actcaagtac ttgaactttt acataaactc 5160
   gttccattac gtggaacccc aaaacgacga ccaaacgaca ctccaaccta tttacctaca 5220
   caggtctacc gacctaccct tttgatagat acttgtgctg tttgtccaaa cttggctatt 5280
   tttacggcta ctatgtactc ggtgcggacg cggactgtat atggtaaatg tggctgctga 5340
   ctactgaaat tagctaattg atgagagcac cctttttcaa acgttataga actgagataa 5400
   tgggttttta tttaccaccc gaattcgaga cacagggtaa gtccgtaata ggaaacccca 5460
   gaaacttacc caccaggtac ctatcctacc aagtcgctca tcggtctaaa tacctactca 5520
   cccggtacct atctattgtt tataagtgtg cagggctcca atgcgtaggc atgttcccaa 5580
   cgttagcaat gggcaagcca acttttaggt gggttctatc acgtcgcctt gtttaaagtg 5640
   aaatgtagat caacatgtct tggagagtcc gaaactgtgg ttgaaagacg gacaattctc 5700
   cgatgttgcg ggaggatcgt acaaagtaaa agcacctgat tcctggagct gtaaatggga 5760
   gtcaggacgt tttgcaagga aaagggttgt tgttgttgtt tgtgtctagg aagcgaatat 5820
   tcaggggtgg aaggtcgaat attacagatc cgcccagtgt ttggaggatc tggacttcgc 5880
   ctagcaggag gagccatgcg cagtgggggc aatgcctgtt gtgaaacggc ctggtgatga 5940
   acaactgccc cacgcgttga ggagagagag tcctttttcg tctttatcac cggttcgcgt 6000
   gggaaaaggt ggagaaacag ctcgcacctg gaccgacctt gagcaaggag cacgcagtat 6060
   gtcacaattg atctcaaaca cacatcattt ccgaagaatt gccagtggta tatctcttgc 6120
   tgcacaacag atggagattc gatcacggtg tgtaagagga ttatcaacat agccacttta 6180
   tcatagcaat attcaatgca cctatatcgc accgggtgat ttggatccag gggagctagc 6240
   attgctggga acggagcctc cagtcttttt ggtccttgtt gccagccttt tttttcgtct 6300
   cttttcgtca ttcatcgctt tctcttcccg gaaaagcatc gaagaatgta cagtagtatg 6360
   cgggagtaga tggtaggacg gcatctccgc agagacatcg gcagccaagt aggttgtgga 6420
   tatccatcga tcttcgttac tttttctgga agctctccat atcaagcggt actgtagtgt 6480
   ttttttttct tttcttttct                                             6500
<210> 2
   <211> 2301
   <212> DNA
   <213> Magnaporthe grisea
<220>
   <221> CDS
   <222> (1)..(2301)
<400> 2
   atg gtt caa tca gcg att ctc ggt ttc ccc cgc atg ggt gtg aac cgt      48
   Met Val Gln Ser Ala Ile Leu Gly Phe Pro Arg Met Gly Val Asn Arg
   1               5                   10                  15
   gac ctg aag aag gcc act gaa gct tac tgg gcc ggc aag atc tcg cag      96
   Asp Leu Lys Lys Ala Thr Glu Ala Tyr Trp Ala Gly Lys Ile Ser Gln
               20                  25                  30
   gac gac ctc ctt gcc gag gct aag agg ctc aga ctc gcc cac tgg aag     144
   Asp Asp Leu Leu Ala Glu Ala Lys Arg Leu Arg Leu Ala His Trp Lys
           35                  40                  45
   atc cag aag gat gcc ggt gtc gac atc atc ccc agc aat gat ttt gct     192
   Ile Gln Lys Asp Ala Gly Val Asp Ile Ile Pro Ser Asn Asp Phe Ala
       50                  55                  60
   ttg tac gac cag gtc ctt tca cac atc cag gac ttc ggt gcc gtt ccc     240
   Leu Tyr Asp Gln Val Leu Ser His Ile Gln Asp Phe Gly Ala Val Pro
   65 70 75 80
   gaa agg tac tca agc tcc aag ctg aac ccc gtc gac gag tac ttc gcc 288
   Glu Arg Tyr Ser Ser Ser Lys Leu Asn Pro Val Asp Glu Tyr Phe Ala
   85 90 95
   atg ggt cgt ggt cac cag aag gat ggt gtc gat gtt ccc agc ttg gag 336
   Met Gly Arg Gly His Gln Lys Asp Gly Val Asp Val Pro Ser Leu Glu
   100 105 110
   atg gtc aag tgg ttc gac tca aac tac cac tac gtc aag ccc act ctc 384
   Met Val Lys Trp Phe Asp Ser Asn Tyr His Tyr Val Lys Pro Thr Leu
   115 120 125
   cag gac aac cag acc ttc acg ctt acg gcc aac ccc aag gct gtg aat 432
   Gln Asp Asn Gln Thr Phe Thr Leu Thr Ala Asn Pro Lys Ala Val Asn
   130 135 140
   gag ttc aac gag gcc aag gag gct ggc atc aac acc cgc ccc gtc ctc 480
   Glu Phe Asn Glu Ala Lys Glu Ala Gly Ile Asn Thr Arg Pro Val Leu
   145 150 155 160
   gtt ggt ccc gtt tct ttc ctt cac ctc gcc aag gct gac cgt ggt cag 528
   Val Gly Pro Val Ser Phe Leu His Leu Ala Lys Ala Asp Arg Gly Gln
   165 170 175
   tct gtt gac ccc atc gac ctc ctt gac aag ctt gtc ccc gtt tac gag 576
   Ser Val Asp Pro Ile Asp Leu Leu Asp Lys Leu Val Pro Val Tyr Glu
   180 185 190
   gag ctc ctc gcc aag ctc aag gcc gct ggt gcc gag act gtc cag att 624
   Glu Leu Leu Ala Lys Leu Lys Ala Ala Gly Ala Glu Thr Val Gln Ile
   195 200 205
   gac gag cct gtc ctc gtc ttc gac ctt ccc gcc aag gtc aag gct gct 672
   Asp Glu Pro Val Leu Val Phe Asp Leu Pro Ala Lys Val Lys Ala Ala
   210 215 220
   ttc aag ccc aca tat gag aag ttt gcc agc ctg ggt gac aag atc ccc 720
   Phe Lys Pro Thr Tyr Glu Lys Phe Ala Ser Leu Gly Asp Lys Ile Pro
   225 230 235 240
   aag ctc gtt ttc gcc aca tac ttc ggt gac atc gtc cac aat ctt gac 768
   Lys Leu Val Phe Ala Thr Tyr Phe Gly Asp Ile Val His Asn Leu Asp
   245 250 255
   ctc gtc ccc aag gac gtc tac gcc gtc cac gtc gac ctc gtc agg aac 816
   Leu Val Pro Lys Asp Val Tyr Ala Val His Val Asp Leu Val Arg Asn
   260 265 270
   cct gag cag ttg gaa act gtt gtt ggt gcc ctg ggc ccc aag acc att 864
   Pro Glu Gln Leu Glu Thr Val Val Gly Ala Leu Gly Pro Lys Thr Ile
   275 280 285
   ctt tct gct ggt atc gtc gat ggc cgt aac atc tgg aag acc aac ttc 912
   Leu Ser Ala Gly Ile Val Asp Gly Arg Asn Ile Trp Lys Thr Asn Phe
   290 295 300
   cag aag gcc att gag act gtt gag agt gcg atc cag aag ctc ggc aag 960
   Gln Lys Ala Ile Glu Thr Val Glu Ser Ala Ile Gln Lys Leu Gly Lys
   305 310 315 320
   gag cgt gtc att gtt gcc act tcc agc tct ctc ctt cac act ccc cac 1008
   Glu Arg Val Ile Val Ala Thr Ser Ser Ser Leu Leu His Thr Pro His
   325 330 335
   aca cta gcg agc gag aag aag ctt gac cct gaa atc gcc gac tgg ttc 1056
   Thr Leu Ala Ser Glu Lys Lys Leu Asp Pro Glu Ile Ala Asp Trp Phe
                   340                 345                 350
   tca ttt gcc tct gag aag gcc gtc gag gtt gcc atc atc gcc aag gcc      1104
   Ser Phe Ala Ser Glu Lys Ala Val Glu Val Ala Ile Ile Ala Lys Ala
           355                 360                 365
   gtc act gag ggc cct gct gct gtc cgc gag cag ctc gag gcc aac gcc      1152
   Val Thr Glu Gly Pro Ala Ala Val Arg Glu Gln Leu Glu Ala Asn Ala
       370                 375                 380
   aag tcg atg aac gct cgc gcc acc tcg agc aga aca aat gac ccc aag      1200
   Lys Ser Met Asn Ala Arg Ala Thr Ser Ser Arg Thr Asn Asp Pro Lys
   385                 390                 395                 400
   gtc aag gag agg cag tca aag att gtc gag tca gac tac aac cgc aag      1248
   Val Lys Glu Arg Gln Ser Lys Ile Val Glu Ser Asp Tyr Asn Arg Lys
                   405                 410                 415
   tcg gag ttc cct acc cgt att tcg cag cag cag gcc aag ctt aac ctt      1296
   Ser Glu Phe Pro Thr Arg Ile Ser Gln Gln Gln Ala Lys Leu Asn Leu
               420                 425                 430
   cct ctc ttt ccc act act acc atc ggg tcc ttc ccc cag acc cag act      1344
   Pro Leu Phe Pro Thr Thr Thr Ile Gly Ser Phe Pro Gln Thr Gln Thr
           435                 440                 445
   atc cgc gcc cag cgt gcc aag ctc acc aag aag gaa att gac gct gag      1392
   Ile Arg Ala Gln Arg Ala Lys Leu Thr Lys Lys Glu Ile Asp Ala Glu
       450                 455                 460
   caa tac gcc aag ttc atc gag gag gag att gag aac aat gta aag atc      1440
   Gln Tyr Ala Lys Phe Ile Glu Glu Glu Ile Glu Asn Asn Val Lys Ile
   465                 470                 475                 480
   cag gaa gag ctc ggt ctg gat gtc ttc gtc cac ggt gag ccc gag cgt      1488
   Gln Glu Glu Leu Gly Leu Asp Val Phe Val His Gly Glu Pro Glu Arg
                   485                 490                 495
   aac gac atg gtg caa ttc ttt ggt gag cgc ctg gac ggt tat gcc ttc      1536
   Asn Asp Met Val Gln Phe Phe Gly Glu Arg Leu Asp Gly Tyr Ala Phe
               500                 505                 510
   acc acg cac gcc tgg gtt cag agc tac ggt tcc cgc tgc gtc cgt cct      1584
   Thr Thr His Ala Trp Val Gln Ser Tyr Gly Ser Arg Cys Val Arg Pro
           515                 520                 525
   ccc atc att gtc ggt gac atc tct cgc ccg gca ccg atg act gtc aag      1632
   Pro Ile Ile Val Gly Asp Ile Ser Arg Pro Ala Pro Met Thr Val Lys
       530                 535                 540
   gaa tca agg tac gct gtc gag att tcc aag aag ccc atg aag ggt atg      1680
   Glu Ser Arg Tyr Ala Val Glu Ile Ser Lys Lys Pro Met Lys Gly Met
   545                 550                 555                 560
   ttg acg ggc ccc gtc acc tgc ctg agg tgg tcg ttc ccc cgt gac gat      1728
   Leu Thr Gly Pro Val Thr Cys Leu Arg Trp Ser Phe Pro Arg Asp Asp
                   565                 570                 575
   gtg cac cag tcc gtc caa gct gag cag ctc gct ctt gct ctc cgt gac      1776
   Val His Gln Ser Val Gln Ala Glu Gln Leu Ala Leu Ala Leu Arg Asp
               580                 585                 590
   gag gtt gtt gac ctt gag aag gct ggt gtc gac gtc atc cag gtc gac      1824
   Glu Val Val Asp Leu Glu Lys Ala Gly Val Asp Val Ile Gln Val Asp
           595                 600                 605
   gag cct gct ctc cgt gag ggt ctg ccc ctc cgc tct ggt aag gag cgc      1872
   Glu Pro Ala Leu Arg Glu Gly Leu Pro Leu Arg Ser Gly Lys Glu Arg
               610                     615                     620
   gat gct tac ctc cag tgg gct gtc aag gct ttc aag ctc tcg acc tgt        1920
   Asp Ala Tyr Leu Gln Trp Ala Val Lys Ala Phe Lys Leu Ser Thr Cys
   625                     630                     635                     640
   ggt gtc gag gac tcg act cag atc cac tcg cac ttc tgc tac tct gag        1968
   Gly Val Glu Asp Ser Thr Gln Ile His Ser His Phe Cys Tyr Ser Glu
                   645                     650                     655
   ttc cag gac ttc ttc cac gcc att gct gcc ctt gat gcc gac gtt ctg        2016
   Phe Gln Asp Phe Phe His Ala Ile Ala Ala Leu Asp Ala Asp Val Leu
               660                     665                     670
   tcc atc gag aac agc aag tct gat gcc aag ctg ctg aag gtg ttc gtc        2064
   Ser Ile Glu Asn Ser Lys Ser Asp Ala Lys Leu Leu Lys Val Phe Val
           675                     680                     685
   gac tcg gct tac ccc cgc cac atc ggc ccc ggt gtc tac gac atc cac        2112
   Asp Ser Ala Tyr Pro Arg His Ile Gly Pro Gly Val Tyr Asp Ile His
       690                     695                     700
   tcc ccc cgt gtt ccc agc gaa cag gag atc aag gac cgc atc gag gag        2160
   Ser Pro Arg Val Pro Ser Glu Gln Glu Ile Lys Asp Arg Ile Glu Glu
   705                     710                     715                     720
   atg ctt cag tac ctc aag cct gag cag ctc tgg atc gac cct gac tgc        2208
   Met Leu Gln Tyr Leu Lys Pro Glu Gln Leu Trp Ile Asp Pro Asp Cys
                   725                     730                     735
   ggt ctg aag acc cgc cag tgg aag gag acc aag gag gct ctc acc aac        2256
   Gly Leu Lys Thr Arg Gln Trp Lys Glu Thr Lys Glu Ala Leu Thr Asn
               740                     745                     750
   atg gtc aac gcc gcc aag tac ttc cgt gcc aag tac gcc aaa taa            2301
   Met Val Asn Ala Ala Lys Tyr Phe Arg Ala Lys Tyr Ala Lys
           755                     760                     765
<210> 3
   <211> 766
   <212> PRT
   <213> Magnaporthe grisea
<400> 3
   Met Val Gln Ser Ala Ile Leu Gly Phe Pro Arg Met Gly Val Asn Arg
   1               5                   10                  15
   Asp Leu Lys Lys Ala Thr Glu Ala Tyr Trp Ala Gly Lys Ile Ser Gln
               20                  25                  30
   Asp Asp Leu Leu Ala Glu Ala Lys Arg Leu Arg Leu Ala His Trp Lys
           35                  40                  45
   Ile Gln Lys Asp Ala Gly Val Asp Ile Ile Pro Ser Asn Asp Phe Ala
       50                  55                  60
   Leu Tyr Asp Gln Val Leu Ser His Ile Gln Asp Phe Gly Ala Val Pro
   65                  70                  75                  80
   Glu Arg Tyr Ser Ser Ser Lys Leu Asn Pro Val Asp Glu Tyr Phe Ala
                   85                  90                  95
   Met Gly Arg Gly His Gln Lys Asp Gly Val Asp Val Pro Ser Leu Glu
   100 105 110
   Met Val Lys Trp Phe Asp Ser Asn Tyr His Tyr Val Lys Pro Thr Leu
   115 120 125
   Gln Asp Asn Gln Thr Phe Thr Leu Thr Ala Asn Pro Lys Ala Val Asn
   130 135 140
   Glu Phe Asn Glu Ala Lys Glu Ala Gly Ile Asn Thr Arg Pro Val Leu
   145 150 155 160
   Val Gly Pro Val Ser Phe Leu His Leu Ala Lys Ala Asp Arg Gly Gln
   165 170 175
   Ser Val Asp Pro Ile Asp Leu Leu Asp Lys Leu Val Pro Val Tyr Glu
   180 185 190
   Glu Leu Leu Ala Lys Leu Lys Ala Ala Gly Ala Glu Thr Val Gln Ile
   195 200 205
   Asp Glu Pro Val Leu Val Phe Asp Leu Pro Ala Lys Val Lys Ala Ala
   210 215 220
   Phe Lys Pro Thr Tyr Glu Lys Phe Ala Ser Leu Gly Asp Lys Ile Pro
   225 230 235 240
   Lys Leu Val Phe Ala Thr Tyr Phe Gly Asp Ile Val His Asn Leu Asp
   245 250 255
   Leu Val Pro Lys Asp Val Tyr Ala Val His Val Asp Leu Val Arg Asn
   260 265 270
   Pro Glu Gln Leu Glu Thr Val Val Gly Ala Leu Gly Pro Lys Thr Ile
   275 280 285
   Leu Ser Ala Gly Ile Val Asp Gly Arg Asn Ile Trp Lys Thr Asn Phe
   290 295 300
   Gln Lys Ala Ile Glu Thr Val Glu Ser Ala Ile Gln Lys Leu Gly Lys
   305 310 315 320
   Glu Arg Val Ile Val Ala Thr Ser Ser Ser Leu Leu His Thr Pro His
   325 330 335
   Thr Leu Ala Ser Glu Lys Lys Leu Asp Pro Glu Ile Ala Asp Trp Phe
   340 345 350
   Ser Phe Ala Ser Glu Lys Ala Val Glu Val Ala Ile Ile Ala Lys Ala
   355 360 365
   Val Thr Glu Gly Pro Ala Ala Val Arg Glu Gln Leu Glu Ala Asn Ala
   370 375 380
   Lys Ser Met Asn Ala Arg Ala Thr Ser Ser Arg Thr Asn Asp Pro Lys
   385 390 395 400
   Val Lys Glu Arg Gln Ser Lys Ile Val Glu Ser Asp Tyr Asn Arg Lys
   405 410 415
   Ser Glu Phe Pro Thr Arg Ile Ser Gln Gln Gln Ala Lys Leu Asn Leu
   420 425 430
   Pro Leu Phe Pro Thr Thr Thr Ile Gly Ser Phe Pro Gln Thr Gln Thr
   435 440 445
   Ile Arg Ala Gln Arg Ala Lys Leu Thr Lys Lys Glu Ile Asp Ala Glu
   450 455 460
   Gln Tyr Ala Lys Phe Ile Glu Glu Glu Ile Glu Asn Asn Val Lys Ile
   465 470 475 480
   Gln Glu Glu Leu Gly Leu Asp Val Phe Val His Gly Glu Pro Glu Arg
   485 490 495
   Asn Asp Met Val Gln Phe Phe Gly Glu Arg Leu Asp Gly Tyr Ala Phe
   500 505 510
   Thr Thr His Ala Trp Val Gln Ser Tyr Gly Ser Arg Cys Val Arg Pro
   515 520 525
   Pro Ile Ile Val Gly Asp Ile Ser Arg Pro Ala Pro Met Thr Val Lys
   530 535 540
   Glu Ser Arg Tyr Ala Val Glu Ile Ser Lys Lys Pro Met Lys Gly Met
   545 550 555 560
   Leu Thr Gly Pro Val Thr Cys Leu Arg Trp Ser Phe Pro Arg Asp Asp
   565 570 575
   Val His Gln Ser Val Gln Ala Glu Gln Leu Ala Leu Ala Leu Arg Asp
   580 585 590
   Glu Val Val Asp Leu Glu Lys Ala Gly Val Asp Val Ile Gln Val Asp
   595 600 605
   Glu Pro Ala Leu Arg Glu Gly Leu Pro Leu Arg Ser Gly Lys Glu Arg
   610 615 620
   Asp Ala Tyr Leu Gln Trp Ala Val Lys Ala Phe Lys Leu Ser Thr Cys
   625 630 635 640
   Gly Val Glu Asp Ser Thr Gln Ile His Ser His Phe Cys Tyr Ser Glu
   645 650 655
   Phe Gln Asp Phe Phe His Ala Ile Ala Ala Leu Asp Ala Asp Val Leu
   660 665 670
   Ser Ile Glu Asn Ser Lys Ser Asp Ala Lys Leu Leu Lys Val Phe Val
   675 680 685
   Asp Ser Ala Tyr Pro Arg His Ile Gly Pro Gly Val Tyr Asp Ile His
   690 695 700
   Ser Pro Arg Val Pro Ser Glu Gln Glu Ile Lys Asp Arg Ile Glu Glu
   705 710 715 720
   Met Leu Gln Tyr Leu Lys Pro Glu Gln Leu Trp Ile Asp Pro Asp Cys
   725 730 735
   Gly Leu Lys Thr Arg Gln Trp Lys Glu Thr Lys Glu Ala Leu Thr Asn
   740 745 750
   Met Val Asn Ala Ala Lys Tyr Phe Arg Ala Lys Tyr Ala Lys
   755 760 765
<210> 4
   <211> 20
   <212> DNA
   <213> Artificial
<220>
   <223> PCR primer
<220>
   <221> amorce
   <222> (1)..(20)
<400> 4 gacagtctgc aatcggaggc 20
<210> 5
   <211> 21
   <212> DNA
   <213> Artificial
<220>
   <223> PCR primer
<220>
   <221> amorce
   <222> (1)..(20)
<400> 5
   gcatggctcc tcctgctagg c 21
<210> 6
   <211> 20
   <212> DNA
   <213> Artificial
<220>
   <223> PCR primer
<220>
   <221> amorce
   <222> (1)..(20)
<400> 6 tagagtagat gccgaccggg 20
<210> 7
   <211> 20
   <212> DNA
   <213> Artificial
<220> <223> PCR primer
<220>
   <221> amorce
   <222> (1)..(20)
<400> 7
   ggctccgttc ccagcaatgc 20
<210> 8
   <211> 20
   <212> DNA
   <213> Artificial
<220>
   <223> PCR primer
<220>
   <221> amorce
   <222> (1)..(20)
<400> 8
   ggagttgctc aagatatcgc 20
<210> 9
   <211> 24
   <212> DNA
   <213> Artificial
<220>
   <223> PCR primer
<400> 9
   aggctctcgc tgaactcccc aatg 24
<210> 10
   <211> 20
   <212> DNA
   <213> Artificial
<220>
   <223> PCR primer
<400> 10
   ggttcaatca gcgattctcg 20
<210> 11
   <211> 20
   <212> DNA
   <213> Artificial
<220>
   <223> PCR primer
<220>
   <221> amorce
   <222> (1)..(20)
<400> 11
   gcttatttgg cgtacttggc 20
<210> 12
   <211> 30
   <212> DNA
   <213> Artificial
<220>
   <223> PCR primer
<220>
   <221> amorce
   <222> (1)..(30)
<400> 12
   ccggaattct gacagtctgc aatcggaggc 30
<210> 13
   <211> 30
   <212> DNA
   <213> Artificial
<220>
   <223> PCR primer
<220>
   <221> amorce
   <222> (1)..(30)
<400> 13
   tccccgcggt ggacggcttc ggtgactggg 30
<210> 14
   <211> 30
   <212> DNA
   <213> Artificial
<220>
   <223> PCR primer
<400> 14
   ggaagatcta gtgcaagcga taagtctccg 30
<210> 15
   <211> 30
   <212> DNA
   <213> Artificial
<220>
   <223> PCR primer
<400> 15
   gcgggatccg catggctcct cctgctaggc 30
<210> 16
   <211> 4570
   <212> DNA
   <213> Ustilago maydis
<220>
   <221> gene
   <222> (1)..(4570)
<400> 16
   tcttgcgttg tatgttgatg atgagcgtgt tccgagaaga acaaatcgag ctatctacgg    60
   tctctctgta tttcaccgct gaagtccaag tacaggcttg tactgtgata actaggctgt   120
   gcgctgcagt gcatgcgtgt gaaccaacac aggctggcca cgcttgtgtg ccctgtgcat   180
   gtgtgtgtct gccgaatcac gaatgctaaa tgcatgtgat tttccagaaa agtgtcactt   240
   tccgaataat cacaaggacg cgcctcttgc tgacataatc aagttcaagg aaattaaagt   300
   taagtcacaa gtcacagtca cgagtgcgag ccgccgaaag cggccaaaca catgtcatcg   360
   ttcataacta gtttccttca cgcacgagtt aaatcaggct tgtagatggc attatccatg   420
   ttgaaatctg gaattgctag gatcatgttg aatgcttttg aggcgggata tcatgttgaa   480
   tgcttacaga atacagaggt ggaatacgag aaacagatgc gaggagagag aaagaaaaag   540
   acaatgacga caatagaggg agaccataaa ctcatactag tgggagatgg gaggggatag   600
   agctgcactc ggcgtagacc aaagcagttg atgctggatc catgggcacg agaagagctt   660
   gttcaagctg gggtgatatg gaaagagaat tttaagcaag ggtctggcgg cactcctcag   720
   cggcagcaac catgttggtg agctgggcag tgcactcctc ccaggttcgg gtcttgagac   780
   cacagtcggg gttgacccag atcgagtcct tgggaagcac ctcggccatg gccttgatgc   840
   ggcccaccat ctcgtccttg ctgggcacac ggggcgagtg gatgtcaaag acaccagggc   900
   caatgtgcga ggggtagcca accttcttga aggcaccgag aaggtgctca ccggacttgg   960
   agtgctcaat cgagatcata tcggcatcga gctcgatgat cgacttcata atgagcgaga  1020
   agtccgagta gcagaagtgc gaggcgatgt tcatggcgtc cgagcaaccc gaagtggaga  1080
   gacggaacga gtcgacagcc catcgcaggt aaccagccca gtccttctgg cggagcggca  1140
   gaccctcacg aatggcgggc tcgtcgacct gaacggcacg aacaccggcc ttctcgaggt  1200
   cgatgacctc gtcgcggagg gcgagggcga tctgcttgga ctgtacctcc ttgctgatgt  1260
   cggcacgagg gaacgaccag ttgaggatgg tgacgggacc agtaagcata cccttcatgg  1320
   gcttcttggt gaggctctga gcgtacgacg accagcgcac agtcatggga gcaggacggc 1380
   tgacgtccga gacgacaacg gggggacgaa cgtatcgcga accgaacgac tggacccagg 1440
   cgttctgggt gaagacgaaa ccgtcgagca gctcaccgaa gtactggacc atgtcgttac 1500
   gctcgggctc accgtgaacg agcacgtcga ggttgagggc ctcctgcttc tcgacgacca 1560
   tcttgatctc gtcttcgagg aacttttcgt actcctcctt ggtgatctcg gacttgttga 1620
   aacgcgcacg gtactgacgg atctccttgg tctgggggaa agagccgatg gtggtggtgg 1680
   ggaagatggg gagcgcaagg tgctccttct gaacctcctt tcgcacgttg aaaggcgact 1740
   tgcgggcgag gtcctcctcc ttgatgttgg caacgcgttc acgaacggcg gggtcagagt 1800
   tcttctcaaa gtcgcgacgg gccttgatgc tcttctcgtt gacggcgaga gcgtcggcgg 1860
   ccgagcttgg gtcgcggaga gcagcagcga gggtggcaat ctcagcacac ttctcgttgg 1920
   cgaacgagaa ccagtcgagc acctcagcgc tcagcttctt ctcgttggcg atggtgatgg 1980
   gggtgtggag gagcgaggac gaagaagcaa tctgaacacg cgaagcgtcg ccgagcttct 2040
   cgacggcctt ctgggcaatc ttgagggcag cgctgaggtc agttttccag atgttacggc 2100
   cggagacgag accgagcgag acgacgtgct tggtgttaac gaaggcggcg aggacctcgt 2160
   cgagctgctc aggagcacgg tcaagatcga tgtggagacc agcgacgggg agggtcttga 2220
   cgatctcgag gttggactcg agcttgttga agtaggtagc aatcatgatc ttgaccgagg 2280
   gggcagcctg agcgatggtc tcgtaggcgg ccttgaactc ggcggcgtac tgctgggcac 2340
   ggtcaagaac gaggacgggc tcatcaatct ggacccactc ggcaccggcc tcgccgagtt 2400
   tggcgagcaa ttcaccgtag acgggggcaa gcttgctgag gagcgagacg gggtcgagct 2460
   cggcgtcctt ggcgtccttg ccgggcttac caagggcgag gagggtgacg ggaccgacga 2520
   ggacggggcg ggcgttgtag ccggcctcct tggcctcaac aaagtcgtcg atgggcttgg 2580
   tgttgttaat cttgaactcg gtggactcgc tcagttcggg gacgaggtag tggtagttcg 2640
   agtcgaacca cttctgcatt tcggtggcgg gaaggtcgac accgttcttc tggtggccac 2700
   gggccatggc gaagtaggtg tcgagagcat caaggccaga cttggcgtag ttctctggaa 2760
   taaggttgaa ggtgttggag gcgtccagga cgtggtcgta gagcgagaag gtaccggtgg 2820
   gcacaacatc gacaccagca tccttgatga acttgtaggt ctggagacgc tgctccttgg 2880
   cgaccttgag gagctcctcc tgggaagact tacctcccca gtaggcctca agagccttct 2940
   tgacctcacg ctgaggacca atgcgggggt aaccgaggac ggcggaagta ctgtgagtgt 3000
   cgagagggag aatagaaata atcagcgaat tgtcttgaaa tcaatgacac gatcatgatg 3060
   agccatgtgc ggttggtact tacgccattt taaatatatg agaggaacga acgaataagg 3120
   gaaagggaat gatagcgaga gacgtctaga acagaggatt cagacgacag tgaacaaact 3180
   tctcgggtcg tggatacgag caagaagtga gaaagaagcg gagatggagg atggtggttg 3240
   ggtgggatgg atgatgtagg aggaagggaa gaaggaaggg gtacttgcgc caggaagaag 3300
   aaataggaat tggcgagcgt gcgaaccatc acttctatgt ggtgtcgtgc tagggcccgg 3360
   tcaggaagga aatcctgcgt tcttctaacc tgtaggcgaa tgagggcgtg cggtagacgt 3420
   tgggcagctg cacagtcgcc actctggtag gcagaaaaag aacagcggct gttggcccaa 3480
   gcgagcaacc taaatgggct gtcgtgcgtt gaggcagagc aaatgcgtgc tcttcgtaac 3540
   cgcacgtttt catccaatcg acaagtgagc agatttttca cttttctgtt tgctgtcgtc 3600
   ttttcttcca gctagctttt cctctgccaa cgtgcctgtc tcaatttttt ttcttgtgag 3660
   ttctgctgcg ctcgcgttgt ggctgccttt ctttggcctt gacggaatct caagcgtttc 3720
   agggtacact gagactgtga ccgtgactct ctgtgactta ggaaggtgtc gtctgccaac 3780
   cagccctgca gctcccgtcc aaaaggcgtg atggcccaac caattcgagt cagtcacgag 3840
   tgtgcgcccc aggtttttgc atcgcatctg atcttttcaa acgttctggt tggtcgagac 3900
   ggctccatgt ctgcagcgcc gcgcggatca ttcacacttt gctatgatag gacaattgtg 3960
   aatcactcgt gactgcaatc gttgattggt ccgaaaccct ctctctcatt cacgtacgaa 4020
   tcgtgaagtg aatctgcacg tacagaagct cttcccctct cttctgttcg aagtctttgc 4080
   tgaggctcct gttgccatca tcactacgat ggggaccgct ggtcgtgcca tctcgaggtg 4140
   tagtaatagg gctgcccgta tgcatcacag atcagcttgt tgcctgaaca gcgcagcgac 4200
   gttcgactca ggtgtctggg tcagcgtgcg ttggcagcct ctcgtgcacg tttatgtccc 4260
   tgttcaactt gtcgagtcat gagcaaactc aggaccgaac atggaaaaat gctgctgttg 4320
   ttggaataga gcgtctaaca atcacgaatg gaggcacagc tgctgcgtgt aggcagtcgt 4380
   gagtcacgag tcgtgagtcg ttgagcctcg tgccttgagg tcacgagtaa cgtcatgttg 4440
   tgttagaccc tggcaaagac gcgatttcaa tttcaatttc gacacctgtt gtccgtgcat 4500
   tcacgattcg tgattgacgc tccttgacgt cttggctggt cactgcgtgt caaactgtga 4560
   ctgagctaga 4570
<210> 17
   <211> 2304
   <212> DNA
   <213> Ustilago maydis
<220>
   <221> CDS
   <222> (1)..(2304)
   <223> cDNA
<400> 17
   atg gct act tcc gcc gtc ctc ggt tac ccc cgc att ggt cct cag cgt      48
   Met Ala Thr Ser Ala Val Leu Gly Tyr Pro Arg Ile Gly Pro Gln Arg
   1               5                   10                  15
   gag gtc aag aag gct ctt gag gcc tac tgg gga ggt aag tct tcc cag      96
   Glu Val Lys Lys Ala Leu Glu Ala Tyr Trp Gly Gly Lys Ser Ser Gln
               20                  25                  30
   gag gag ctc ctc aag gtc gcc aag gag cag cgt ctc cag acc tac aag     144
   Glu Glu Leu Leu Lys Val Ala Lys Glu Gln Arg Leu Gln Thr Tyr Lys
           35                  40                  45
   ttc atc aag gat gct ggt gtc gat gtt gtg ccc acc ggt acc ttc tcg      192
   Phe Ile Lys Asp Ala Gly Val Asp Val Val Pro Thr Gly Thr Phe Ser
       50                  55                  60
   ctc tac gac cac gtc ctg gac gcc tcc aac acc ttc aac ctt att cca      240
   Leu Tyr Asp His Val Leu Asp Ala Ser Asn Thr Phe Asn Leu Ile Pro
   65                  70                  75                  80
   gag aac tac gcc aag tct ggc ctt gat gct ctc gac acc tac ttc gcc      288
   Glu Asn Tyr Ala Lys Ser Gly Leu Asp Ala Leu Asp Thr Tyr Phe Ala
                   85                  90                  95
   atg gcc cgt ggc cac cag aag aac ggt gtc gac ctt ccc gcc acc gaa      336
   Met Ala Arg Gly His Gln Lys Asn Gly Val Asp Leu Pro Ala Thr Glu
               100                 105                 110
   atg cag aag tgg ttc gac tcg aac tac cac tac ctc gtc ccc gaa ctg      384
   Met Gln Lys Trp Phe Asp Ser Asn Tyr His Tyr Leu Val Pro Glu Leu
           115                 120                 125
   agc gag tcc acc gag ttc aag att aac aac acc aag ccc atc gac gac      432
   Ser Glu Ser Thr Glu Phe Lys Ile Asn Asn Thr Lys Pro Ile Asp Asp
       130                 135                 140
   ttt gtt gag gcc aag gag gcc ggc tac aac gcc cgc ccc gtc ctc gtc      480
   Phe Val Glu Ala Lys Glu Ala Gly Tyr Asn Ala Arg Pro Val Leu Val
   145                 150                 155                 160
   ggt ccc gtc acc ctc ctc gcc ctt ggt aag ccc ggc aag gac gcc aag      528
   Gly Pro Val Thr Leu Leu Ala Leu Gly Lys Pro Gly Lys Asp Ala Lys
                   165                 170                 175
   gac gcc gag ctc gac ccc gtc tcg ctc ctc agc aag ctt gcc ccc gtc      576
   Asp Ala Glu Leu Asp Pro Val Ser Leu Leu Ser Lys Leu Ala Pro Val
               180                 185                 190
   tac ggt gaa ttg ctc gcc aaa ctc ggc gag gcc ggt gcc gag tgg gtc      624
   Tyr Gly Glu Leu Leu Ala Lys Leu Gly Glu Ala Gly Ala Glu Trp Val
           195                 200                 205
   cag att gat gag ccc gtc ctc gtt ctt gac cgt gcc cag cag tac gcc      672
   Gln Ile Asp Glu Pro Val Leu Val Leu Asp Arg Ala Gln Gln Tyr Ala
       210                 215                 220
   gcc gag ttc aag gcc gcc tac gag acc atc gct cag gct gcc ccc tcg      720
   Ala Glu Phe Lys Ala Ala Tyr Glu Thr Ile Ala Gln Ala Ala Pro Ser
   225                 230                 235                 240
   gtc aag atc atg att gct acc tac ttc aac aag ctc gag tcc aac ctc      768
   Val Lys Ile Met Ile Ala Thr Tyr Phe Asn Lys Leu Glu Ser Asn Leu
                   245                 250                 255
   gag atc gtc aag acc ctc ccc gtc gct ggt ctc cac atc gat ctt gac      816
   Glu Ile Val Lys Thr Leu Pro Val Ala Gly Leu His Ile Asp Leu Asp
               260                 265                 270
   cgt gct cct gag cag ctc gac gag gtc ctc gcc gcc ttc gtt aac acc      864
   Arg Ala Pro Glu Gln Leu Asp Glu Val Leu Ala Ala Phe Val Asn Thr
           275                 280                 285
   aag cac gtc gtc tcg ctc ggt ctc gtc tcc ggc cgt aac atc tgg aaa      912
   Lys His Val Val Ser Leu Gly Leu Val Ser Gly Arg Asn Ile Trp Lys
       290                 295                 300
   act gac ctc agc gct gcc ctc aag att gcc cag aag gcc gtc gag aag      960
   Thr Asp Leu Ser Ala Ala Leu Lys Ile Ala Gln Lys Ala Val Glu Lys
   305                 310                 315                 320
   ctc ggc gac gct tcg cgt gtt cag att gct tct tcg tcc tcg ctc ctc 1008
   Leu Gly Asp Ala Ser Arg Val Gln Ile Ala Ser Ser Ser Ser Leu Leu
   325 330 335
   cac acc ccc atc acc atc gcc aac gag aag aag ctg agc gct gag gtg 1056
   His Thr Pro Ile Thr Ile Ala Asn Glu Lys Lys Leu Ser Ala Glu Val
   340 345 350
   ctc gac tgg ttc tcg ttc gcc aac gag aag tgt gct gag att gcc acc 1104
   Leu Asp Trp Phe Ser Phe Ala Asn Glu Lys Cys Ala Glu Ile Ala Thr
   355 360 365
   ctc gct gct gct ctc cgc gac cca agc tcg gcc gcc gac gct ctc gcc 1152
   Leu Ala Ala Ala Leu Arg Asp Pro Ser Ser Ala Ala Asp Ala Leu Ala
   370 375 380
   gtc aac gag aag agc atc aag gcc cgt cgc gac ttt gag aag aac tct 1200
   Val Asn Glu Lys Ser Ile Lys Ala Arg Arg Asp Phe Glu Lys Asn Ser
   385 390 395 400
   gac ccc gcc gtt cgt gaa cgc gtt gcc aac atc aag gag gag gac ctc 1248
   Asp Pro Ala Val Arg Glu Arg Val Ala Asn Ile Lys Glu Glu Asp Leu
   405 410 415
   gcc cgc aag tcg cct ttc aac gtg cga aag gag gtt cag aag gag cac 1296
   Ala Arg Lys Ser Pro Phe Asn Val Arg Lys Glu Val Gln Lys Glu His
   420 425 430
   ctt gcg ctc ccc atc ttc ccc acc acc acc atc ggc tct ttc ccc cag 1344
   Leu Ala Leu Pro Ile Phe Pro Thr Thr Thr Ile Gly Ser Phe Pro Gln
   435 440 445
   acc aag gag atc cgt cag tac cgt gcg cgt ttc aac aag tcc gag atc 1392
   Thr Lys Glu Ile Arg Gln Tyr Arg Ala Arg Phe Asn Lys Ser Glu Ile
   450 455 460
   acc aag gag gag tac gaa aag ttc ctc gaa gac gag atc aag atg gtc 1440
   Thr Lys Glu Glu Tyr Glu Lys Phe Leu Glu Asp Glu Ile Lys Met Val
   465 470 475 480
   gtc gag aag cag gag gcc ctc aac ctc gac gtg ctc gtt cac ggt gag 1488
   Val Glu Lys Gln Glu Ala Leu Asn Leu Asp Val Leu Val His Gly Glu
   485 490 495
   ccc gag cgt aac gac atg gtc cag tac ttc ggt gag ctg ctc gac ggt 1536
   Pro Glu Arg Asn Asp Met Val Gln Tyr Phe Gly Glu Leu Leu Asp Gly
   500 505 510
   ttc gtc ttc acc cag aac gcc tgg gtc cag tcg ttc ggt tcg cga tac 1584
   Phe Val Phe Thr Gln Asn Ala Trp Val Gln Ser Phe Gly Ser Arg Tyr
   515 520 525
   gtt cgt ccc ccc gtt gtc gtc tcg gac gtc agc cgt cct gct ccc atg 1632
   Val Arg Pro Pro Val Val Val Ser Asp Val Ser Arg Pro Ala Pro Met
   530 535 540
   act gtg cgc tgg tcg tcg tac gct cag agc ctc acc aag aag ccc atg 1680
   Thr Val Arg Trp Ser Ser Tyr Ala Gln Ser Leu Thr Lys Lys Pro Met
   545 550 555 560
   aag ggt atg ctt act ggt ccc gtc acc atc ctc aac tgg tcg ttc cct 1728
   Lys Gly Met Leu Thr Gly Pro Val Thr Ile Leu Asn Trp Ser Phe Pro
   565 570 575
   cgt gcc gac atc agc aag gag gta cag tcc aag cag atc gcc ctc gcc 1776
   Arg Ala Asp Ile Ser Lys Glu Val Gln Ser Lys Gln Ile Ala Leu Ala
   580 585 590
   ctc cgc gac gag gtc atc gac ctc gag aag gcc ggt gtt cgt gcc gtt      1824
   Leu Arg Asp Glu Val Ile Asp Leu Glu Lys Ala Gly Val Arg Ala Val
           595                 600                 605
   cag gtc gac gag ccc gcc att cgt gag ggt ctg ccg ctc cgc cag aag      1872
   Gln Val Asp Glu Pro Ala Ile Arg Glu Gly Leu Pro Leu Arg Gln Lys
       610                 615                 620
   gac tgg gct ggt tac ctg cga tgg gct gtc gac tcg ttc cgt ctc tcc      1920
   Asp Trp Ala Gly Tyr Leu Arg Trp Ala Val Asp Ser Phe Arg Leu Ser
   625                 630                 635                 640
   act tcg ggt tgc tcg gac gcc atg aac atc gcc tcg cac ttc tgc tac      1968
   Thr Ser Gly Cys Ser Asp Ala Met Asn Ile Ala Ser His Phe Cys Tyr
                   645                 650                 655
   tcg gac ttc tcg ctc att atg aag tcg atc atc gag ctc gat gcc gat      2016
   Ser Asp Phe Ser Leu Ile Met Lys Ser Ile Ile Glu Leu Asp Ala Asp
               660                 665                 670
   atg atc tcg att gag cac tcc aag tcc ggt gag cac ctt ctc ggt gcc      2064
   Met Ile Ser Ile Glu His Ser Lys Ser Gly Glu His Leu Leu Gly Ala
           675                 680                 685
   ttc aag aag gtt ggc tac ccc tcg cac att ggc cct ggt gtc ttt gac      2112
   Phe Lys Lys Val Gly Tyr Pro Ser His Ile Gly Pro Gly Val Phe Asp
       690                 695                 700
   atc cac tcg ccc cgt gtg ccc agc aag gac gag atg gtg ggc cgc atc      2160
   Ile His Ser Pro Arg Val Pro Ser Lys Asp Glu Met Val Gly Arg Ile
   705                 710                 715                 720
   aag gcc atg gcc gag gtg ctt ccc aag gac tcg atc tgg gtc aac ccc      2208
   Lys Ala Met Ala Glu Val Leu Pro Lys Asp Ser Ile Trp Val Asn Pro
                   725                 730                 735
   gac tgt ggt ctc aag acc cga acc tgg gag gag tgc act gcc cag ctc      2256
   Asp Cys Gly Leu Lys Thr Arg Thr Trp Glu Glu Cys Thr Ala Gln Leu
               740                 745                 750
   acc aac atg gtt gct gcc gct gag gag tgc cgc cag acc ctt gct taa      2304
   Thr Asn Met Val Ala Ala Ala Glu Glu Cys Arg Gln Thr Leu Ala
           755                 760                 765
<210> 18
   <211> 767
   <212> PRT
   <213> ustilago maydis
<400> 18
   Met Ala Thr Ser Ala Val Leu Gly Tyr Pro Arg Ile Gly Pro Gln Arg
   1               5                   10                  15
   Glu Val Lys Lys Ala Leu Glu Ala Tyr Trp Gly Gly Lys Ser Ser Gln
               20                  25                  30
   Glu Glu Leu Leu Lys Val Ala Lys Glu Gln Arg Leu Gln Thr Tyr Lys
           35                  40                  45
   Phe Ile Lys Asp Ala Gly Val Asp Val Val Pro Thr Gly Thr Phe Ser
       50                  55                  60
   Leu Tyr Asp His Val Leu Asp Ala Ser Asn Thr Phe Asn Leu Ile Pro
   65                  70                  75                  80
   Glu Asn Tyr Ala Lys Ser Gly Leu Asp Ala Leu Asp Thr Tyr Phe Ala
                   85                  90                  95
   Met Ala Arg Gly His Gln Lys Asn Gly Val Asp Leu Pro Ala Thr Glu
               100                 105                 110
   Met Gln Lys Trp Phe Asp Ser Asn Tyr His Tyr Leu Val Pro Glu Leu
           115                 120                 125
   Ser Glu Ser Thr Glu Phe Lys Ile Asn Asn Thr Lys Pro Ile Asp Asp
       130                 135                 140
   Phe Val Glu Ala Lys Glu Ala Gly Tyr Asn Ala Arg Pro Val Leu Val
   145                 150                 155                 160
   Gly Pro Val Thr Leu Leu Ala Leu Gly Lys Pro Gly Lys Asp Ala Lys
                   165                 170                 175
   Asp Ala Glu Leu Asp Pro Val Ser Leu Leu Ser Lys Leu Ala Pro Val
               180                 185                 190
   Tyr Gly Glu Leu Leu Ala Lys Leu Gly Glu Ala Gly Ala Glu Trp Val
           195                 200                 205
   Gln Ile Asp Glu Pro Val Leu Val Leu Asp Arg Ala Gln Gln Tyr Ala
       210                 215                 220
   Ala Glu Phe Lys Ala Ala Tyr Glu Thr Ile Ala Gln Ala Ala Pro Ser
   225                 230                 235                 240
   Val Lys Ile Met Ile Ala Thr Tyr Phe Asn Lys Leu Glu Ser Asn Leu
                   245                 250                 255
   Glu Ile Val Lys Thr Leu Pro Val Ala Gly Leu His Ile Asp Leu Asp
               260                 265                 270
   Arg Ala Pro Glu Gln Leu Asp Glu Val Leu Ala Ala Phe Val Asn Thr
           275                 280                 285
   Lys His Val Val Ser Leu Gly Leu Val Ser Gly Arg Asn Ile Trp Lys
       290                 295                 300
   Thr Asp Leu Ser Ala Ala Leu Lys Ile Ala Gln Lys Ala Val Glu Lys
   305                 310                 315                 320
   Leu Gly Asp Ala Ser Arg Val Gln Ile Ala Ser Ser Ser Ser Leu Leu
                   325                 330                 335
   His Thr Pro Ile Thr Ile Ala Asn Glu Lys Lys Leu Ser Ala Glu Val
               340                 345                 350
   Leu Asp Trp Phe Ser Phe Ala Asn Glu Lys Cys Ala Glu Ile Ala Thr
           355                 360                 365
   Leu Ala Ala Ala Leu Arg Asp Pro Ser Ser Ala Ala Asp Ala Leu Ala
       370                 375                 380
   Val Asn Glu Lys Ser Ile Lys Ala Arg Arg Asp Phe Glu Lys Asn Ser
   385                 390                 395                 400
   Asp Pro Ala Val Arg Glu Arg Val Ala Asn Ile Lys Glu Glu Asp Leu
                   405                 410                 415
   Ala Arg Lys Ser Pro Phe Asn Val Arg Lys Glu Val Gln Lys Glu His
               420                 425                 430
   Leu Ala Leu Pro Ile Phe Pro Thr Thr Thr Ile Gly Ser Phe Pro Gln
           435                 440                 445
   Thr Lys Glu Ile Arg Gln Tyr Arg Ala Arg Phe Asn Lys Ser Glu Ile
       450                 455                 460
   Thr Lys Glu Glu Tyr Glu Lys Phe Leu Glu Asp Glu Ile Lys Met Val
   465                 470                 475                 480
   Val Glu Lys Gln Glu Ala Leu Asn Leu Asp Val Leu Val His Gly Glu
                   485                 490                 495
   Pro Glu Arg Asn Asp Met Val Gln Tyr Phe Gly Glu Leu Leu Asp Gly
               500                 505                 510
   Phe Val Phe Thr Gln Asn Ala Trp Val Gln Ser Phe Gly Ser Arg Tyr
           515                 520                 525
   Val Arg Pro Pro Val Val Val Ser Asp Val Ser Arg Pro Ala Pro Met
       530                 535                 540
   Thr Val Arg Trp Ser Ser Tyr Ala Gln Ser Leu Thr Lys Lys Pro Met
   545                 550                 555                 560
   Lys Gly Met Leu Thr Gly Pro Val Thr Ile Leu Asn Trp Ser Phe Pro
                   565                 570                 575
   Arg Ala Asp Ile Ser Lys Glu Val Gln Ser Lys Gln Ile Ala Leu Ala
               580                 585                 590
   Leu Arg Asp Glu Val Ile Asp Leu Glu Lys Ala Gly Val Arg Ala Val
           595                 600                 605
   Gln Val Asp Glu Pro Ala Ile Arg Glu Gly Leu Pro Leu Arg Gln Lys
       610                 615                 620
   Asp Trp Ala Gly Tyr Leu Arg Trp Ala Val Asp Ser Phe Arg Leu Ser
   625                 630                 635                 640
   Thr Ser Gly Cys Ser Asp Ala Met Asn Ile Ala Ser His Phe Cys Tyr
                   645                 650                 655
   Ser Asp Phe Ser Leu Ile Met Lys Ser Ile Ile Glu Leu Asp Ala Asp
               660                 665                 670
   Met Ile Ser Ile Glu His Ser Lys Ser Gly Glu His Leu Leu Gly Ala
           675                 680                 685
   Phe Lys Lys Val Gly Tyr Pro Ser His Ile Gly Pro Gly Val Phe Asp
       690                 695                 700
   Ile His Ser Pro Arg Val Pro Ser Lys Asp Glu Met Val Gly Arg Ile
   705                 710                 715                 720
   Lys Ala Met Ala Glu Val Leu Pro Lys Asp Ser Ile Trp Val Asn Pro
                   725                 730                 735
   Asp Cys Gly Leu Lys Thr Arg Thr Trp Glu Glu Cys Thr Ala Gln Leu
               740                 745                 750
   Thr Asn Met Val Ala Ala Ala Glu Glu Cys Arg Gln Thr Leu Ala
           755                 760                 765
<210> 19
   <211> 1003
   <212> DNA
   <213> Phytophthora infestans
<220>
   <221> EST
   <222> (1)..(1003)
<220>
   <221> misc_feature
   <222> (970)..(970)
   <223> n is a, c, g, or t
<400> 19
   cggacgcgtg gkctggcgcg cacttctcaa cgcaagtccg tcccagttca gtaaaccttc      60
   tctacacata tatcaagatg gtcgccgtcg acagtgcaac gctgggtttc ccccgcatgg     120
   gccccaaccg tgagctcaag ttcgcccttg agaagttctg gcgcaacaag attagcgagg     180
   aggagctgta caagattgct aactctgtgg aggaggccaa ctggaagaag cagatcgacg     240
   ccggtgtcag ccgtgtgggt gttggcctct tctcgctcta cgaccacgtg ctggactgga     300
   cctactactt ggatctggcc ccagagcgct tcgcgtcggt gcccgcmggt ctgtcgcagt     360
   acttcgctat ggcccgtggc gttgacggca tccccgctct tgacatgacg aagtggttcg     420
   actccaacta ccactacgag gtgcctgagc tcaacgccaa gtccacgccc aaggctaact     480
   tcggctcgta tgtggccagc atcaagcgcg ctttggctgt tgtgggcccc aacaagacgg     540
   tcccgatcat tctcggccct ctgacttacc tggccctgag caagtacgac ggcgccaccc     600
   ttgacgagtt gctggtcaag gtcctgcccc tctacactgc tctgctgaac gagctcgccg     660
   gtctaggtgt gcaggaggtg caggtgcacg agccttccct cgttggcact caggccgatc     720
   agttggccaa gcaccttgcc accgtctacg gatccaagga ccagaagggt gccattcagc     780
   acgagaagct cgccattaac ctggctacat actttgagga gatcaaccac gacgtgtacc     840
   agtggtttgc cacgtcgcct gtgtcggcca tctcgcttga ctttacccgc ggtgacaacc     900
   tgtcggtgct acagaagttc ggcttccttg ccggcaagcg tctcggtgcc ggtctgatcg     960
   acggccctan cgtgtggaag tttcaacccc gacacgatct gtc                      1003
<210> 20
   <211> 308
   <212> PRT
   <213> Phytophthora infestans
<220>
   <221> Proteine
   <222> (1)..(308)
<400> 20
   Met Val Ala Val Asp Ser Ala Thr Leu Gly Phe Pro Arg Met Gly Pro
   1               5                   10                  15
   Asn Arg Glu Leu Lys Phe Ala Leu Glu Lys Phe Trp Arg Asn Lys Ile
               20                  25                  30
   Ser Glu Glu Glu Leu Tyr Lys Ile Ala Asn Ser Val Glu Glu Ala Asn
           35                  40                  45
   Trp Lys Lys Gln Ile Asp Ala Gly Val Ser Arg Val Gly Val Gly Leu
       50                  55                  60
   Phe Ser Leu Tyr Asp His Val Leu Asp Trp Thr Tyr Tyr Leu Asp Leu
   65                  70                  75                  80
   Ala Pro Glu Arg Phe Ala Ser Val Pro Ala Gly Leu Ser Gln Tyr Phe
                   85                  90                  95
   Ala Met Ala Arg Gly Val Asp Gly Ile Pro Ala Leu Asp Met Thr Lys
               100                 105                 110
   Trp Phe Asp Ser Asn Tyr His Tyr Glu Val Pro Glu Leu Asn Ala Lys
           115                 120                 125
   Ser Thr Pro Lys Ala Asn Phe Gly Ser Tyr Val Ala Ser Ile Lys Arg
   130 135 140
   Ala Leu Ala Val Val Gly Pro Asn Lys Thr Val Pro Ile Ile Leu Gly
   145 150 155 160
   Pro Leu Thr Tyr Leu Ala Leu Ser Lys Tyr Asp Gly Ala Thr Leu Asp
   165 170 175
   Glu Leu Leu Val Lys Val Leu Pro Leu Tyr Thr Ala Leu Leu Asn Glu
   180 185 190
   Leu Ala Gly Leu Gly Val Gln Glu Val Gln Val His Glu Pro Ser Leu
   195 200 205
   Val Gly Thr Gln Ala Asp Gln Leu Ala Lys His Leu Ala Thr Val Tyr
   210 215 220
   Gly Ser Lys Asp Gln Lys Gly Ala Ile Gln His Glu Lys Leu Ala Ile
   225 230 235 240
   Asn Leu Ala Thr Tyr Phe Glu Glu Ile Asn His Asp Val Tyr Gln Trp
   245 250 255
   Phe Ala Thr Ser Pro Val Ser Ala Ile Ser Leu Asp Phe Thr Arg Gly
   260 265 270
   Asp Asn Leu Ser Val Leu Gln Lys Phe Gly Phe Leu Ala Gly Lys Arg
   275 280 285
   Leu Gly Ala Gly Leu Ile Asp Gly Pro Xaa Val Trp Lys Phe Gln Pro
   290 295 300
   Arg His Asp Leu
   305

## Description

La présente invention concerne l'utilisation d'inhibiteurs de méthionine synthase pour le traitement des maladies fongiques et plus particulièrement le traitement des maladies fongiques des espèces végétales cultivées.

Les champignons sont responsables d'épidémies dévastatrices qui peuvent entraîner des pertes importantes au niveau des cultures de différentes espèces végétales. Le principe d'employer des inhibiteurs d'enzymes de champignons pathogènes, et d'utiliser ces enzymes dans des tests pour identifier de nouvelles molécules actives contre ces champignons sont connues en soi. Toutefois, la simple caractérisation d'une enzyme de champignon n'est pas suffisante pour atteindre cet objectif, encore faut-il que l'enzyme choisie comme cible de molécules antifongiques potentielles soit essentielle à la vie du champignon, son inhibition par la molécule antifongique entraînant la mort du champignon ou essentielle à la pathogénie du champignon, son inhibition n'étant pas létale pour le champignon mais inhibant simplement son pouvoir pathogène. L'identification de voies métaboliques et d'enzymes indispensables à la pathogénie et à la survie du champignon est donc nécessaire au développement de nouveaux produits antifongiques.

La voie d'assimilation du soufre comprend l'incorporation de l'ion sulfate (SO₄²⁻), son activation, et sa réduction en soufre réduit (S²⁻). Ces étapes sont catalysées successivement par une ATP sulfurylase (EC 2.7.7.4), une APS kinase (EC 2.7.1.25), une PAPS réductase (EC 1.8.4.8) (APS réductase chez les organismes photosynthétiques, EC 1.8.4.9), et une (NADPH₂) sulfite réductase (EC 1.8.1.2) (une enzyme dépendante de la ferrédoxine chez les organismes photosynthétiques, EC 1.8.7.1). Chez tous les organismes autotrophes, la voie d'assimilation, d'activation et de réduction de l'ion sulfate est conservée dans son principe général, l'incorporation du soufre réduit au sein d'un squelette carboné présente des variations importantes selon les organismes: bactéries¹ (Ex : *Escherichia coli*), plantes² (Ex : *Arabidopsis thaliana*), levures (Ex: *Saccharomyces cerevisiae³*) et les champignons filamenteux⁴. En effet, chez les plantes et les bactéries, le soufre réduit est incorporé au sein d'une molécule en C3 qui dérive de la sérine, pour former la cystéine. Le soufre est alors transféré sur une molécule en C4 qui dérive de l'homosérine, pour former l'homocystéine. Cette série de réactions forme la voie de transsulfuration directe. A l'inverse, chez *Saccharomyces cerevisiae* (*S. cerevisae*), le soufre est directement incorporé au sein d'une molécule en C4 qui dérive de l'homoserine pour former l'homocystéine (sulphydrylation directe)³. La cystéine est alors synthétisée à partir de l'homocystéine par une série de réactions qui compose la voie de transsulfuration inverse. Chez les champignons filamenteux, la synthèse de l'homocystéine s'effectue à la fois par la voie décrite chez les plantes (transsulfuration directe) et par celle de *S. cerevisiae* (sulphydrylation directe). De plus, la synthèse de la cystéine est réalisée soit par l'intermédiaire de la serine soit à partir de l'homocystéine *via* la voie de transsulfuration inverse. Ces différentes voies métaboliques ont été définies à la suite de la caractérisation de mutants auxotrophes pour la cystéine et pour la méthionine chez *Neurospora crassa* (*N. crassa*)⁵ et *Aspergillus nidulans* (*A. nidulans*)⁶. Ce modèle peut être extrapolé à tous les champignons filamenteux dont les champignons pathogènes des plantes (par exemple *Magnaporthe grisea, M. grisea*) et des animaux (par exemple *Aspergillus fumigatus (A. fumigatus)*).
*M. grisea,* un pathogène de type ascomycète responsable de dégâts importants sur les cultures de riz est un modèle de choix pour une telle approche. La synthèse de la méthionine chez les champignons filamenteux nécessite l'action d'une méthionine synthase de type vitamine B12-indépendante comme chez les plantes. L'approche décrite dans l'étude du gène de la méthionine synthase de *Cryptococcus neoformans*²⁸, un pathogène humain, diffère de la présente invention. En effet, si les animaux (humains compris) sont capables de synthétiser la méthionine, cette étape est catalysée par une méthionine synthase de type vitamine B12-dépendante très différente de celle des autres Eucaryotes comme les plantes et les champignons. La méthionine synthase de plante présente de fortes homologies au niveau protéique avec celle de *M. grisea,* mais présente en outre des différences d'ordre structurales d'après les modélisations réalisées^{9,12,27}. Aussi, l'identification de l'enzyme fongique et sa caractérisation sont nécessaires pour la détermination de ses caractéristiques propres permettant l'identification d'inhibiteurs fongiques uniquement. Le choix et l'application de tels inhibiteurs dans des procédés de traitement des cultures végétales seront alors spécifiques. L'expression de la méthionine synthase d'un champignon phytopathogène a été étudiée (Solomon et al, Mol. Plant Pathol., 2000, 1(5): 315-323). Ainsi, la présente invention décrit que les mutants du gène *MET6* et plus particulièrement les mutants de délétion du gène *MET6* codant pour la méthionine synthase de *M. grisea* sont auxotrophes pour la méthionine et sont non pathogènes. Chez ces mutants, le processus infectieux est fortement affecté au niveau de la phase de pénétration du pathogène dans la cellule végétale mais aussi dans sa capacité à progresser dans les tissus infectés. Le pouvoir pathogène des mutants méthionine synthase de *M. grisea* est partiellement restauré lors de l'ajout de méthionine lors de l'infection. Ces résultats montrent que l'absence d'activité méthionine synthase est fatale au champignon lors de l'infection. Des résultats similaires ont été obtenus chez *Utilago maydis (U. maydis)* et *Phytophtora infestans (P. infestans)*.

### Description de la liste de séquences

SEQ ID No.1 : gène de la méthionine synthase de *Magnaporthe grisea*
SEQ ID No. 2 : ADNc de la méthionine synthase de *Magnaporthe grisea*
SEQ ID No. 3 : séquence protéique de la méthionine synthase de *Magnaporthe grisea*
SEQ ID No. 4 : amorce Met6-5
SEQ ID No. 5 : amorce Met6-6
SEQ ID No. 6 : amorce HphRP10
SEQ ID No. 7 : amorce Met6-7
SEQ ID No. 8 : amorce Met6-10
SEQ ID No. 9 : amorce dCGS-hph-end(-)
SEQ ID No. 10 : amorce Met6-8
SEQ ID No. 11 : amorce Met6-9
SEQ ID No. 12 : amorce Met6-1
SEQ ID No. 13 : amorce Met6-2
SEQ ID No. 14 : amorce Met6-3
SEQ ID No. 15 : amorce Met6-4
SEQ ID No.16 : gène de la méthionine synthase de *U. maydis*
SEQ ID No. 17 : ADNc de la méthionine synthase de *U. maydis*
SEQ ID No. 18 : séquence protéique de la méthionine synthase de *U. maydis*
SEQ ID No.19 : séquence EST de la méthionine synthase de *P. infestans*
SEQ ID No. 20 : séquence protéique déduite de la méthionine synthase de *P. infestans*

### Description de l'invention

La présente invention a pour objet un procédé pour l'identification de composés antifongiques pour le traitement des maladies fongiques des espèces végétales cultivées comprenant l'identification de composés inhibant l'activité enzymatique de la méthionine synthase d'un champignon phytopathogène.

De tels procédés sont connus avec d'autres enzymes cibles (par exemple US 2004/191,849).

Les maladies fongiques sont définies, dans le cadre de la présente invention, comme des maladies dues à des champignons pathogènes des plantes appartenant aux familles des ascomycètes, basidiomycètes et oomycètes.

Les composés antifongiques identifiables par le procédé selon l'invention peuvent être utilisés dans un procédé de lutte, à titre curatif ou préventif, contre les champignons phytopathogènes des cultures, caractérisé en ce que l'on applique sur le sol où poussent où sont susceptibles de pousser les végétaux, sur les feuilles et/ou les fruits des végétaux ou sur les semences des végétaux, une quantité efficace (agronomiquement efficace) et non phytotoxique d'un inhibiteur de la méthionine synthase. Par "quantité efficace et non phytotoxique", on entend une quantité d'inhibiteur suffisante pour permettre le contrôle du cycle de développement ou la destruction des champignons présents ou susceptibles d'apparaître sur les cultures, et n'entraînant pour lesdites cultures aucun symptôme notable de phytotoxicité. Une telle quantité est susceptible de varier dans de larges limites selon la famille de champignon à combattre, le type de culture, les conditions climatiques, et les composés compris dans la composition antifongique selon l'invention. Cette quantité peut être déterminée par des essais systématiques au champ, à la portée de l'homme du métier.

Les composés antifongiques identifiables par le procédé selon l'invention sont utiles pour traiter les semences de céréales (blé, seigle, triticale et orge notamment), de pomme de terre, de coton, de pois, de colza, de maïs, de lin ou encore les semences d'arbres forestiers ou encore les semences génétiquement modifiées de ces végétaux. La présente invention concerne aussi l'application foliaire sur les cultures végétales, c'est-à-dire sur les feuillages, les fleurs, les fruits et/ou les troncs des végétaux concernés, mais également tout autre type d'application. Parmi les végétaux visés par les composés identifiables selon l'invention, on peut citer le riz, le maïs, le coton, les céréales, comme le blé, l'orge, le triticale, les arbres fruitiers, en particulier pommiers, poiriers, pêchers, vigne, bananiers, orangers, citronniers, etc..., les cultures oléagineuses, par exemple, colza, tournesol, les cultures maraîchères et légumières, tomates, salades, les cultures protéagineuses, le pois, les solanées, par exemple la pomme de terre, la betterave, le lin, et les arbres forestiers, ainsi que les homologues génétiquement modifiés de ces cultures.

Parmi les végétaux visés par les composés identifiables selon l'invention, on peut citer
- le blé, en ce qui concerne la lutte contre les maladies suivantes des semences : les fusarioses *(Microdochium nivale* et *Fusarium roseum),* les caries *(Tilletia caries, Tilletia controversa* ou *Tilletia indica),* la septoriose *(Septoria nodorum);* le charbon nu *(Ustilago tritici)*;
- le blé, en ce qui concerne la lutte contre les maladies suivantes des parties aériennes de la plante : le piétin-verse (Tapesia *yallundae, Tapesia acuiformis)*, le piétin-échaudage *(Gaeumannomyces graminis)*, la fusariose du pied *(F. culmorum, F. graminearum)*, la fusariose des épis *(F. culmorum, F. graminearum, Microdochium nivale),* le rhizoctone *(Rhizoctonia cerealis)*, l'oïdium *(Erysiphe graminis forma specie tritici),* les rouilles *(Puccinia striiformis* et *Puccinia recondita)* et les septorioses *(Septoria tritici et Septoria nodorum),* l'helminthosporiose *(Drechslera tritici-repentis) ;*
- l'orge, en ce qui concerne la lutte contre les maladies suivantes des semences : les helminthosporioses *(Pyrenophora graminea, Pyrenophora teres et Cochliobolus sativus*), le charbon nu *(Ustilago nuda)* et les fusarioses *(Microdochium nivale* et *Fusarium roseum);*
- l'orge, en ce qui concerne la lutte contre les maladies suivantes des parties aériennes de la plante : le piétin-verse *(Tapesia yallundae), les helminthosporioses (Pyrenophora teres et Cochtiobolus sativus)*, l'oïdium *(Erysiphe graminis forma specie hordei), la rouille naine (Puccinia hordei)* et la rhynchosporiose *(Rhynchosporium secalis);*
- la pomme de terre, en ce qui concerne la lutte contre les maladies du tubercule (notamment *Helminthosporium solani, Phoma tuberosa, Rhizoctonia solani, Fusarium solani)*, et le le mildiou *(Phytopthora infestans) ;*
- la pomme de terre en ce qui concerne la lutte contre les maladies du feuillage suivantes : l'alternariose *(Alternaria solani)*, le mildiou *(Phytophthora infestans);*
- le coton, en ce qui concerne la lutte contre les maladies suivantes des plantules issues des semences : les fontes de semis et les nécroses du collet *(Rhizoctonia solani, Fusarium oxysporum)*, la pourriture noire des racines *(Thielaviopsis basicola)*;
- les cultures protéagineuses, par exemple le pois, en ce qui concerne la lutte contre les maladies suivantes des semences : l'anthracnose *(Ascochyta pisi, Mycosphaerella pinodes),* la fusariose *(Fusarium oxysporum)*, la pourriture grise *(Botrytis cinerea)*, le mildiou *(Peronospora pisi);*
- les cultures oléagineuses, par exemple le colza, en ce qui concerne la lutte contre les maladies suivantes des semences : *Phoma lingam, Alternaria brassicae et Sclerotinia sclerotiorum;*
- le maïs, en ce qui concerne la lutte contre les maladies des semences : *(Rhizopus sp., Penicillium sp., Trichoderma sp., Aspergillus sp. Et Gibberella fujikuroi);*
- le lin, en ce qui concerne la lutte contre la maladie des semences *:A*/*temaria linicola;*
- les arbres forestiers, en ce qui concerne la lutte contre les fontes de semis *(Fusarium oxysporum, Rhizoctonia solani);*
- le riz en ce qui concerne la lutte contre les maladies suivantes des parties aériennes : la pyriculariose *(Magnaporthe grisea),* le rhizoctone *(Rhizoctonia solani) ;*
- les cultures légumières en ce qui concerne la lutte contre les maladies suivantes des semis ou des jeunes plants issus de semences : les fontes de semis et les nécroses du collet *(Fusarium oxysporum, Fusarium roseum, Rhizoctonia solani, Pythium sp.);*
- les cultures légumières en ce qui concerne la lutte contre les maladies suivantes des parties aériennes : la pourriture grise *(Botrytis sp.)*, les oïdiums *(notamment Erysiphe cichoracearum, Sphaerotheca fuliginea, Leveillula taurica)*, les fusarioses *(Fusarium oxysporum, Fusarium roseum),* les cladosporioses *(C*/*adosporium sp.)*, les alternarioses *(Alternaria sp.),* les anthracnoses *(Colletotrichum sp.),* les septorioses *(Septoria sp.), le rhizoctone (Rhizoctonia solani)*, les mildious (par exemple *Bremia lactucae, Peronospora sp., Pseudoperonospora sp, Phytophthora sp);*
- les arbres fruitiers en ce qui concerne les maladies des parties aériennes : la moniliose *(Monilia fructigenae, M.* /*axa)*, la tavelure *(Venturia inaequalis)*, l'oïdium *(Podosphaera leucotricha);*
- la vigne en ce qui concerne les maladies du feuillage : notamment la pourriture grise *(Botrytis cinerea),* l'oïdium *(Uncinula necator),* le black-rot *(Guignardia biwelli),* le mildiou *(Plasmopara viticola);*
- la betterave en ce qui concerne les maladies suivantes des parties aériennes : la cercosporiose *(Cercospora beticola),* l'oïdium *(Erysiphe beticola),* la ramulariose *(Ramularia beticola)*.

La méthionine synthase est une enzyme bien caractérisée que l'on retrouve chez les plantes et les microorganismes (bactéries, levures, champignons).

De préférence, la méthionine synthase est isolée, purifiée ou partiellement purifiée de son environnement naturel. La méthionine synthase peut être préparée au moyen de différents procédés. Ces procédés sont notamment la purification à partir de sources naturelles telles que des cellules exprimant naturellement ces polypeptides, la production de polypeptides recombinants par des cellules hôtes appropriées et leur purification ultérieure, la production par synthèse chimique ou, enfin, une combinaison de ces différentes approches. Ces différents procédés de production sont bien connus de l'homme du métier.

Dans l'un des modes de réalisation de l'invention, la méthionine synthase est purifiée à partir d'un organisme produisant naturellement cette enzyme comme par exemple les bactéries telle que *E. coli,* les levures telles que *S. cerevisiae*, ou les champignons tels que *N. crassa* ou *M.grisea*.

Dans un mode de réalisation préféré de l'invention, la méthionine synthase est surexprimée dans un organisme hôte recombinant. Les méthodes d'ingénierie des fragments d'ADN et l'expression de polypeptides dans des cellules hôtes sont bien connues de l'homme du métier et ont par exemple été décrites dans "Current Protocols in Molecular Biology" Volumes 1 et 2, Ausubel F.M. et al , publiés par Greene Publishing Associates et Wiley -Interscience (1989) ou dans Molecular Cloning, T.Maniatis, E.F.Fritsch, J.Sambrook (1982).

Dans un mode de réalisation particulier de l'invention, les inhibiteurs de la méthionine synthase inhibent la méthionine synthase de *M. grisea*, représentée par une séquence comprenant l'identifiant de séquence SEQ ID No. 3, de *U. maydis* plus particulièrement représentée par une séquence comprenant l'identifiant de séquence SEQ ID No. 18 ou encore de *P. infestans* notamment représentée comprenant l'identifiant de séquence SEQ ID No. 20. Ladite méthionine synthase peut être codée par le gène de *M. grisea* représenté par une séquence comprenant l'identifiant de séquence SEQ ID No. 1 ou par l'ADNc représenté par une séquence comprenant l'identifiant de séquence SEQ ID No. 2, par le gène de *U. maydis* représenté par une séquence comprenant l'identifiant de séquence SEQ ID No. 16 ou par l'ADNc représenté par une séquence comprenant l'identifiant de séquence SEQ ID No. 17, ou encore par le gène de *P. infectans* représenté par une séquence comprenant l'identifiant de séquence SEQ ID No. 19.

Les composés identifiables par la méthode selon l'invention peuvent avantageusement être mélangés avec d'autres composés antifongiques, notamment avec l'acibenzolar-S-methyl, l'azoxystrobin, le benalaxyl, le benomyl, la blasticidin-S, le bromuconazole, le captafol, le captane, le carbendazim, la carboxine, le carpropamide, le chlorothalonil, les compositions antifongiques à base de cuivre ou de dérivés du cuivre tels que l'hydroxide de cuivre ou l'oxychloride de cuivre, le cyazofamide, le cymoxanil, le cyproconazole, le cyprodinyl, le dichloran, le diclocymet, le dicloran, le diethofencarb, le difenoconazole, le diflumetorim, le dimethomorph, le diniconazole, le discostrobin, le dodemorph, la dodine, l'edifenphos, l'epoxyconazole, l'ethaboxam, l'ethirimol, la famoxadone, la fenamidone, le fenarimol, le fenbuconazole, le fenhexamid, le fenpiclonil, la fenpropidine, le fenpropimorph, la ferimzone, le fluazinam, le fludioxonil, le flumetover, le fluquinconazole, le flusilazole, le flusulfamide, le flutolanil, le flutriafol, le folpet, le furalaxyl, le furametpyr, la guazatine, l'hexaconazole, l'hymexazol, l'imazalil, l'iprobenphos, l'iprodione, l'isoprothiolane, la kasugamycin, le kresoxim-methyl, le mancozeb, le maneb, le mefenoxam, le mepanipyrim, le metalaxyl et ses entiomères tels que le metalaxyl-M, le metconazole, le metiram-zinc, la metominostrobin, l'oxadixyl, le pefurazoate, le penconazole, le pencycuron, l'acide phosporique et ses dérivés tels que le fosetyl-Al, la phthalide, la picoxystrobine, le probenazole, le prochloraz, la procymidone, le propamocarb, le propiconazole, la pyraclostrobin, le pyrimethanil, le pyroquilon, le quinoxyfen, le silthiofam, le simeconazole, la spiroxamine, le tebuconazole, le tetraconazole, le thiabendazole, la thifluzamide, le thiophanate, e.g. thiophanate-methyl, le thiram, le triadimefon, le triadimenol, le tricyclazole, le tridemorph, la trifloxystrobine, le triticonazole, les dérivés de la valinamide tels que par exemple l'iprovalicarb, la vinclozoline, le zineb et le zoxamide. Les mélanges ainsi obtenus ont un spectre d'activité plus large. Les composés identifiables par la méthode selon l'invention peuvent également être associés à un ou plusieurs insecticides, bactéricides, acaricides ou des phéromones ou d'autres composés ayant une activité biologique.

Pour mettre en oeuvre le procédé d'identification de composés antifongiques selon l'invention, la réaction enzymatique est effectuée en présence du composé à tester pour mesurer l'inhibition de l'activité enzymatique de la méthionine synthase. Tous les tests biochimiques permettant de mesurer l'activité enzymatique de la méthionine synthase et donc d'identifier des composés inhibant cette activité enzymatique peuvent être utilisés dans les procédés selon l'invention.

Un essai biochimique à haut débit est proposé afin de cribler des inhibiteurs spécifiques de cette enzyme.

De manière préférée, les procédés d'identification de composés inhibant l'activité de la méthionine synthase comprennent la mise en contact de ces composés avec la méthionine synthase en présence de ses substrats : l'homocystéine, du méthyltetrahydrofolate ou des dérivés polyglutamate du méthyltetrahydrofolate (CH₃-H₄)PteGluₙ) et de différents cofacteurs comme le phosphate, le magnésium et le zinc; et la mesure de l'activité enzymatique.

La mesure de l'activité enzymatique de la méthionine synthase peut être associée à la mesure de la formation de méthionine, de tétrahydrofolate ou encore de méthényltétrahydrofolate ou de tout produit ainsi obtenu, ou encore la mesure de ladite activité par toute autre réaction chimique ou enzymatique.

La mesure de l'activité enzymatique de la méthionine synthase peut également être réalisée en présence d'une enzyme de couplage. La S-adénosylméthionine synthase (AdoMetS) peut être utilisée en tant que telle, elle catalyse la formation de S-adénosylméthionine (S-AdoMet) en présence de la méthionine, d'ATP et de magnésium. La mesure de l'activité enzymatique de la méthionine synthase peut alors être associée à la mesure de la formation du S-adénosylméthionine, du phosphate ou du pyrophosphate.

Selon un autre aspect de l'invention, les procédés d'identification de composés inhibant l'activité enzymatique de la méthionine synthase comprennent l'expression de la méthionine synthase dans un organisme hôte, la purification de la méthionine synthase produite par l'organisme hôte, la mise en contact de ces composés avec la méthionine synthase purifiée et de ses substrats et la mesure de l'activité enzymatique.

Dans un mode de réalisation préféré, tous ces procédés comprennent une étape supplémentaire dans laquelle on détermine si lesdits composés inhibant l'activité enzymatique de la méthionine synthase inhibent la croissance et/ou la pathogénie des champignons.

La présente invention concerne donc des procédés pour identifier des composés inhibant la croissance et/ou la pathogénie des champignons en inhibant l'activité enzymatique de la méthionine synthase. Ces procédés consistant à soumettre un composé, ou un mélange de composés, à un test approprié pour l'identification des composés inhibiteurs de la méthionine synthase et à sélectionner les composés réagissant de manière positive audit test, le cas échéant à les isoler, puis à les identifier.

De manière préférentielle, le test approprié est un test de l'activité enzymatique de la méthionine synthase tel que défini ci-dessus.

De manière préférée, un composé identifié selon ces procédés est ensuite testé pour ces propriétés antifongiques selon des méthodes connues de l'homme du métier. Préférentiellement, le composé est évalué à l'aide de tests phénotypiques tels que des essais de pathogénie sur feuilles détachées ou sur plantes entières.

Par composé on entend selon l'invention tout composé chimique ou mélange de composés chimiques, y compris les peptides et les protéines.

Par mélange de composés on comprend selon l'invention au moins deux composés différents, comme par exemple les (dia)stéréoisomères d'une molécule, des mélanges d'origine naturelle issus de l'extraction de matériel biologique (plantes, tissus végétaux, culture bactériennes, cultures de levures ou de champignons, insectes, tissus animaux, etc.) ou des mélanges réactionnels non purifiés ou purifiés totalement ou en partie, ou encore des mélanges de produits issus de techniques de chimie combinatoire.

De manière préférentielle, les composés inhibiteurs de la pathogénie des champignons inhibant l'activité enzymatique de la méthionine synthase ne sont pas des inhibiteurs généraux d'enzymes. De manière également préférentielle, les composés selon l'invention ne sont pas des composés déjà connus pour avoir une activité antifongique et/ou une activité sur la pathogénie des champignons.

### Exemple 1: Caractérisation du gène de la méthionine synthase chez les champignons.

Le gène de la méthionine synthase a été identifié dans le génome de *M. grisea* version V2 en utilisant la séquence protéique de la méthionine synthase d'A. *nidulans⁷* (NCBI, Accession number: AAF82115) comme modèle. La séquence nucléotidique complète du gène de la méthionine synthase localisé sur le Contig 2.150 (MG_contig_2.150, position 6196-8629, brin complémentaire, SEQ ID No.1) comprend 3 exons correspondant à un cDNA de 2301 bp (SEQ ID No.2) qui code pour un polypeptide de 766 acides aminés (SEQID No.3). La séquence du gène et l'épissage conduisant au messager définitif ont pu être confirmés grâce aux nombreux ESTs identifiées dans les différentes bases publiques et privées. La méthionine synthase de *M. grisea* est codée par un gène unique comme chez A. *nidulans*⁷ L'analyse de la séquence protéique primaire déduite de l'ADNc putatif présente de 48 à 79% d'homologies avec les méthionine synthase indépendante de la vitamine B₁₂ de *S. cerevisae* (P05694), *A. nidulans* (AAF82115), de la bactérie *E. coli* (P13009) et de la plante *A. thaliana* (AAF00639).

La séquence primaire de la méthionine synthase de *M. grisea* présente deux domaines conservés correspondant au domaine méthionine synthase (334 résidus, E = 4e⁻¹¹⁶, pfam01717) caractéristique de cette enzyme. Ce domaine permet la production de méthionine par transfert d'un groupe méthyle du méthyltétrahydrofolate triglutamate sur l'homocystéine. Cette région est localisée dans la partie C-terminale de la protéine. Un second domaine, COG0620 ou Methionine synthase II (méthyltransférase) concerne la partie N-terminale de la protéine (330 acides aminés)⁸. La spécificité de chacun de ces domaines vis-à-vis des substrats de l'enzyme, l'homocystéine, le méthyltétrahydrofolate et du produit de la réaction, la méthionine, a pu être déterminé récemment sur l'enzyme cristallisé de A. *thaliana*⁹

La séquence primaire de la méthionine synthase de *M. grisea*, a été utilisée pour rechercher des orthologues chez les différentes espèces fongiques dont le génome est partiellement ou totalement séquencé. Ces différentes séquences primaires ont été ensuite comparées aux méthionines synthase décrites dans différents organismes comme les plantes, les bactéries et les animaux. La caractérisation de la structure des gènes (introns + exons) et des séquences primaires en acides aminés a été réalisée en utilisant les programmes appropriés (tblastn ; FGENSH; PSI-PHI-BLAST). Selon cette procédure la méthionine synthase a pu être caractérisée chez plusieurs champignons (ascomycètes et basidiomycètes) et un oomycète phytopathogène (*P. sojae et P; infestans*). Un arbre phylogénétique de la méthionine synthase a pu être établi et la représentation obtenue montre que la méthionine synthase de *M. grisea* appartient aux méthionines synthases d'Ascomycètes et qu'elle est éloignée de celles des basidiomycètes. Globalement, l'arbre obtenu est en accord avec celui retraçant l'origine phylogénétique de ces organismes¹⁰.

### Exemple 2 : Délétion du gène de la méthionine synthase de Magnaporthe grisea

L'étude du rôle du gène de la méthionine synthase dans le développement et le processus infectieux de *M. grisea* a été réalisé par l'étude du phénotype des mutants de délétion de ce gène. La stratégie d'obtention de mutants de délétion repose sur le remplacement du gène *MET6* par un allèle mutant dans lequel la phase ouverte de lecture de *MET6* a été remplacée par une cassette de résistance à un antibiotique pour la sélection des transformants (hygromycine).

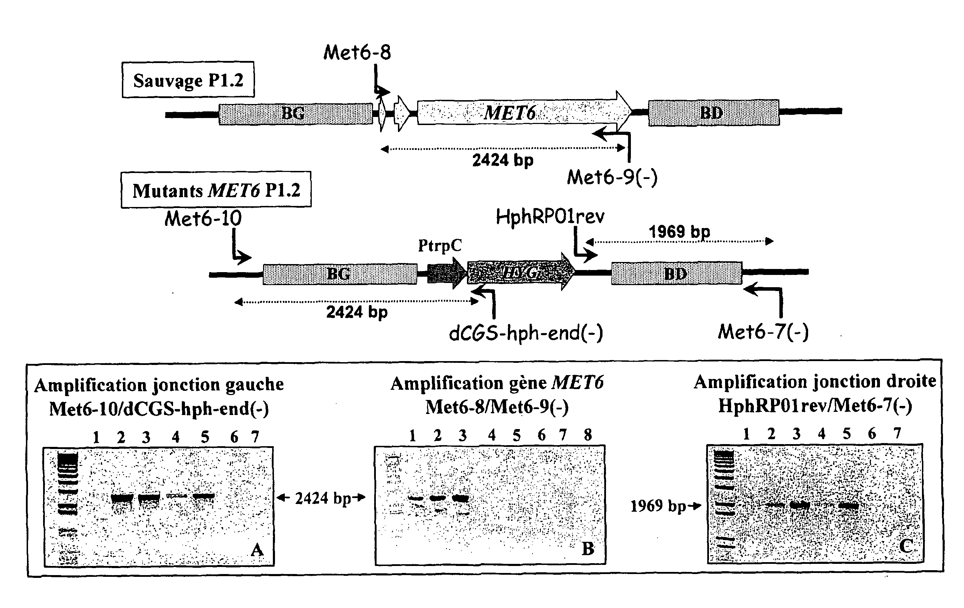

La construction de ce vecteur de remplacement du gène *MET6* de *M. grisea* est réalisée en deux étapes, (i) l'amplification des régions qui bordent ce gène par PCR et correspondant respectivement à des régions génomiques d'environ 1-kb situées de part et d'autre de *MET6.* (ii) la ligation de ces fragments d'ADN génomique à un gène de résistance à un antibiotique permettant de sélectionner les transformants. Ainsi, les fragments de PCR utilisés pour le remplacement du gène sont constitués des deux régions qui sont dites bordure gauche et bordure droite du gène étudié (**Figure 1**). Nous avons choisi le gène de résistance à l'hygromycine (*HYG*, comprenant le promoteur PtrpC, la partie codante à l'hygromycinegène *hph*) comme gène de sélection. La ligation du gène *HYG* est d'abord réalisée *via* les sites *Sac*II / *Bgl*II et les sites *Eco*RI / *Sac*II de la bordure gauche (amorces Met6-1 / Met6-2) du gène *MET6*. La bordure droite du gène *MET6* (amorces Met6-3 / Met6-4) est ensuite introduite *via* le site *Pme*I en aval du gène *hph* (**Figure 1**). Le vecteur de remplacement comprend donc la bordure gauche (BG) du gène *MET6* (région promotrice de 1475 bp), la cassette *HYG* (1400 bp) et la bordure droite (BD) du gène *MET6* (région terminatrice de 1251 bp). Le produit de ligation (BG-hph-BD) a ensuite été cloné dans un vecteur plasmidique. La cassette de remplacement du gène *MET6* a ensuite été amplifiée à partir du plasmide correspondant par PCR avec des amorces spécifiques des extrémités des bordures du gène *MET6* (amorces Met6-5 / Met6-6). Un séquençage des jonctions entre les bordures et la cassette *HYG* du vecteur de remplacement a permis de vérifier la construction. Le produit de PCR (1µg), purifié par électrophorèse en gel d'agarose, est ensuite utilisé pour transformer des protoplastes de la souche sauvage de *M. grisea* P1.2 selon les techniques classiques développées au laboratoire. Les produits issus de la transformation sont sélectionnés sur un milieu contenant l'antibiotique correspondant (Hygromycine).

### Exemple 3: Identification et caractérisation trophique des mutants de délétion met6Δ::hph obtenus par remplacement de gène

Les transformants primaires sont sélectionnés pour leur capacité à se développer en présence d'hygromycine. L'identification des mutants *met6*Δ::*hph* est réalisé en mesurant la croissance différentielle des transformants sur un milieu minimum contenant de l'hygromycine complémenté ou non avec 1 mM méthionine. Les mutants *met6*Δ::*hph* sont incapables de se développer sur ce milieu minimum, mais ils ont une croissance normale sur ce milieu minimum complémenté en méthionine. La fréquence des mutants est de l'ordre de 20% des transformants primaires analysés dans nos conditions expérimentales. Ces mutants ont été ensuite purifiés génétiquement par isolement de monospores.

Les cinq mutants *met6*Δ::*hph* obtenus (4.1, 15.1, 22.1 et 23.1) sont incapables de se développer sur un milieu minimum permettant la croissance de la souche sauvage P1.2. L'addition de méthionine au milieu minimum restaure la croissance des mutants. La complémentation trophique des mutants *met6*Δ::*hph* par l'ajout de méthionine indique que la synthèse de méthionine est affectée par la délétion du gène *MET6*.

L'addition de donneurs de soufre comme la cystéine ou le glutathion (précurseurs de l'homocystéine substrat de la méthionine synthase) ne sont pas suffisants pour restaurer la croissance des mutants *met6*Δ::*hph* de *M.grisea*. En conséquence, il n'existe pas de voie parallèle qui utiliserait le méthyltétrahydrofolate ou ses dérivés poly-glutamate pour la synthèse de la méthionine chez ce champignon. Ainsi la synthèse *de novo* de la méthionine est catalysée uniquement par la méthionine synthase. L'activité de cette enzyme est donc essentielle pour le développement du champignon.

Par contre, en présence de S-adénosylméthionine (SAM ou AdoMet), composé qui dérive directement de la méthionine et qui est indispensable au cycle cellulaire ou, du *S-*méthylméthionine (SMM), composé synthétisée chez les plantes, les mutants *met6*Δ::*hph M. grisea* sont capables de se développer bien qu'avec une croissance réduite par rapport à la souche sauvage P1.2. Le SAM et le SMM sont capables de pénétrer et d'être métabolisé en méthionine par *M. grisea*. Ce mécanisme est probablement similaire à celui décrit chez la levure, *S. cerevisae*. En effet, chez ce champignon ascomycète, le SAM et SMM sont incorporés dans la cellule *via* les transporteurs SAM3 et MPP1, et ensuite transformés en méthionine en présence de l'homocystéine par des homocystéine-S-méthyltransférases (SAM4 et MHT1) qui utilisent respectivement le SAM ou le SMM comme donneur de groupement méthyle (*S. cerevisae*,)¹¹. D'après nos conditions expérimentales, l'addition de SAM (1 mM) au milieu minimum est plus efficace que celle de SMM pour la restauration de la croissance des mutants *met6*Δ::*hph*. Ces résultats suggèrent que *M. grisea* possède des transporteurs et des homocystéines méthyltransférase similaires à ceux décrits chez *S. cerevisae*. Une analyse du génome de *M. grisea* par recherche d'homologies de séquences (tBlastN) vis-à-vis des protéines SAM4 et MHT1 de levure, permet de mettre en évidence un gène orthologue de SAM4 chez *M. grisea*. Il semblerait que les champignons filamenteux ne possèdent qu'un gène (pas d'orthologue de MHT1). D'après nos résultats (meilleure croissance sur SAM que sur SMM), cette protéine pourrait avoir une plus forte affinité pour le SAM que pour le SMM.

Plusieurs transformants dits ectopiques, correspondant à des transformants ayant intégré le vecteur de remplacement BG-hph-BD à un autre locus que celui du gène *MET6* ont été aussi analysés. Ces transformants ectopiques résistants à l'hygromycine sont capables de se développer sur un milieu minimum. Le gène de la méthionine synthase est donc fonctionnel chez ces transformants ectopiques et le vecteur s'est inséré à un locus du génome qui n'a pas d'influence sur le développement du pathogène dans nos conditions expérimentales (capacité à se développer sur le milieu minimum ou complet, sporulation).

### Exemple 4 : Caractérisation moléculaire des mutants met6Δ::hph

Les mutants *met6*Δ::*hph* sont cultivés un milieu contenant de la méthionine (1 mM) afin d'extraire leur ADN génomique qui sera utilisé pour réaliser une analyse moléculaire du locus *MET6* par PCR et par hybridation de type Southern.

L'analyse moléculaire des transformants est réalisée par l'amplification des régions génomiques du locus *MET6* à l'aide des différentes amorces spécifiques du remplacement du de l'allèle sauvage du gène *MET6* de P1.2 par le l'alléle mutant *met6*Δ::*hph*. Ces PCR sont réalisées pour chaque mutant avec des oligonucléotides spécifiques. Les réactions utilisent des oligonucléotides hybridants : d'une part avec le gène de résistance à l'hygromycine *hph* et d'autre part avec une séquence génomique du locus *MET6* située à l'extérieur de la région de *MET6* utilisée pour la construction du vecteur de remplacement (jonction gauche et jonction droite); avec les séquences homologues du gène *MET6* endogène. Ainsi, l'amplification d'un fragment de 1969-bp (jonction gauche) ou de 2447-bp (jonction droite) n'a lieu que dans le cas d'un remplacement du gène sauvage par le gène *met6*Δ*::hph* de la construction (amorces HphRP01rev (SEQ ID No. 6) / Met6-7(-) (SEQ ID No.7) et Met6-10 (SEQ ID No. 8) /dCGS-hph-end(-) (SEQ ID No.9) (Figure 1)). Aucune amplification n'est obtenue dans le cas de la souche sauvage P1.2 ou des transformants ectopiques. De même, l'absence d'amplification du gène *MET6* chez les mutants *met6*Δ::hph (amorces Met6-8, SEQ ID No.10 / Met6-9, SEQ ID No.11) (Figure 1)) est une indication que le gène *MET6* est bien absent chez ces transformants. Par contre, avec ces amorces, un fragment de 2424 bp est amplifié chez la souche sauvage P1.2 et chez les transformants ectopiques. Seules les PCR réalisées avec les mutants 22. 2 et 23.1 donnent les résultats attendus avec ces trois types de PCR. Aucune amplification n'a pu être obtenue, pour des raisons inexpliquées avec les mutants 4.1 et 15.1 dans les PCR Met6-10 (SEQ ID No.8) /dCGS-hph-end (SEQ ID No. 9) et hphRP01 (SEQ ID No.6) / Met6-7(-), SEQ ID No. 7).

Une analyse par hybridation de Southern après digestion de l'ADN génomique par l'enzyme de restriction *BamH*1 a été réalisée et les signaux d'hybridation obtenus pour les des 4 mutants ont été comparés avec ceux obtenus avec la souche sauvage P1.2 et le transformant ectopique (19.1). En utilisant comme sonde le fragment de PCR (Met6-1 (SEQ ID No.12) / Met6-2(SEQ ID No.13)) correspondant à la bordure gauche *MET6* présente dans le vecteur de remplacement (région du promoteur de *MET6*), 2 bandes sont observées pour les mutants dont les tailles sont différentes de celles de la souche sauvage P1.2 et du transformant ectopique (19.1). Le signal correspond à la région promotrice de *MET6* chez la souche sauvage P1.2 et le transformant ectopique 19.1. Ce dernier présente également un signal d'hybridation correspondant au vecteur de remplacement inséré dans une autre région génomique. Un résultat similaire est obtenu en utilisant un fragment PCR (Met6-3 (SEQ ID No. 14) / Met6-4 (SEQ ID No.15)) correspondant à la bordure droite de *MET6* présente dans le vecteur de remplacement (région du terminateur de *MET6*). Avec une sonde spécifique du gène inséré (*hph*), seuls les mutants *met6*Δ::hph et le transformant ectopique 19.1 présentent un signal d'hybridation correspondant soit à la présence de *hph* au locus *MET6* (mutants), soit au vecteur de remplacement BG-*met6*Δ*::hph*-BD (ectopique). Ces derniers résultats indiquent que les différents mutants analysés sont identiques au niveau moléculaire et ne contiennent qu'une seule copie du gène *hph* insérée au locus *MET6* à la place de la phase codante de *MET6.*

### Exemple 5 Analyse du pouvoir pathogène des mutants met6Δ::hph de Magnaporthe grisea

Le pouvoir pathogène des mutants *met6*Δ::*hph* de *M. grisea*, a été évalué à l'aide d'un test d'infection sur des feuilles d'orge en survie et sur des plantes entières d'orge et de riz. Cette analyse a été suivie d'une mesure du taux de germination des spores, de différentiation des appressoria et de pénétration dans des feuilles d'orge. Les spores de la souche sauvage P1.2 et des mutants *met6*Δ::*hph* 4.1, 15.1, 22.1, 23.1 et 24.1 sont récoltées après une croissance de 14 jours sur un milieu à base de farine de riz contenant 1 mM. Le matériel végétal utilisé est l'orge (cv. Express) et le riz (cv. Sariceltik).

Dans les expériences conduites sur des feuilles d'orge en survie, les feuilles sont incubées sur un milieu gélosé (1% gélose- H₂O) contenant de la kinétine (2 mg.ml⁻¹) dans une chambre climatique tempérée (26°C) a forte humidité (100%) et sous une lumière de 100 microeinsteins. Lors de l'infection, le dépôt des spores sur les feuilles est effectué soit sous la forme de gouttelettes (35 □I) ou sous la forme d'un badigeon de la surface des feuilles de la suspension de spores à l'aide d'un coton tige. Ces expériences sont réalisées en absence ou en présence de méthionine 1 mM pendant toute l'incubation ou pendant 24 heures seulement. La concentration en spores est de 3.10⁴ spore/ml à 1.10⁵ spores/ml dans l'eau. L'apparition des symptômes provoqués par *M. grisea* est ensuite observée pendant au moins 7 jours d'incubation. L'inoculation de spores de la souche sauvage de *M. grisea* P1.2 à des feuilles d'orges en survie provoque des nécroses caractéristiques du développement du champignon dans la feuille infectée (lésions sporulantes). A l'opposé, les mutants *met6*Δ:*hph* 4.1, 15.1, 22.1, 23.1 et 24.1 ne provoquent aucun symptôme sur les feuilles d'orge quelque soit le mode d'inoculation et sont donc non pathogènes. L'addition de méthionine aux spores de mutant *met6*Δ::hph permet de restaurer partiellement leur pouvoir pathogène (développement de lésions caractéristiques mais non sporulant). De plus, dans nos conditions expérimentales, nous n'avons pas observé de différences marquées dans les infections réalisées avec des feuilles blessées ou non blessées. Ces observations suggèrent que les mutants *met6*Δ::*hph* sont incapables de pénétrer dans les feuilles d'orge, même blessées. Le jaunissement des feuilles observé lors des expériences réalisées avec étalement des spores par badigeon est probablement dû à un vieillissement prématuré des feuilles dans nos conditions expérimentales. Les résultats sont résumés dans le tableau suivant :

**Tableau I : Estimation du pouvoir pathogène des mutants met6Δ::hph de Magnaporthe grisea vis-à-vis de l'orge**

| **Feuilles d'orge en survie- *Magnaporthe grisea* 3.10⁴ spores/ml** | | |
|---|---|---|
| | **Sans méthionine** | **Avec méthionine 1 mM** |
| | **Souches sauvages** | |
| P1.2 | **+++** | +++ |
| | **Souches mutantes ectopiques** | |
| Ectopique 19.1 | **+++** | +++ |
| Ectopique 20.1 | **+++** | +++ |
| | **Mutants** *met6*Δ::*hph* | |
| 4.1 | 0 | + |
| 15.1 | 0 | + |
| 22.1 | 0 | + |
| 23.1 | 0 | + |

| | | |
|---|---|---|
| Légende : 0 : pas de lésions ; + : nécroses (lésions) non sporulante ; +++ : lésions sporulantes | | |

L'observation et la quantification des différentes étapes de l'infection (germination, formation de l'appressorium et pénétration), sont réalisées à partir d'une inoculation de feuilles d'orges en survie à l'aide de gouttes de 35 □I d'une suspension de spores (3 10⁵ spores/ml). Après 24 heures, l'épiderme de la feuille est pelé afin d'observer au microscope le nombre de spores germées, ayant différencié un appressorium et le nombre d'évènements de pénétration dans la cellule épidermique. Une solution de calcofluor à 0.01% permet de provoquer une intense fluorescence des parois des cellules fongiques situées à la surface de la plante (les hyphes localisés dans la cellule épidermique ne sont pas colorés Ces observations **(Tableau II)** mettent en évidence que les mutants *met6*Δ::*hph* ont une germination légèrement réduite (-10 à -40 % par rapport à la souche sauvage). Leur taux de différentiation appressoriale est aussi légèrement réduit (0 à -30 % par rapport à la souche sauvage). Par contre, ces appressoria mutants sont incapables de pénétrer dans les tissus foliaires.

**Tableau II : Développement des mutants met6Δ::hph de Magnaporthe grisea sur orge**

| **Souches *M. grisea*** | **% Germination** | **% Appressorium / Spores germées** | **% Pénétration** |
|---|---|---|---|
| Δ*MET6*-4.1 | 45 | 52 | 0 |
| Δ*MET6*-15.1 | 70 | 66 | ND |
| Δ*MET6*-22.1 | 62 | 80 | ND |
| Δ*MET6*-23.1 | 37 | 63 | 0 |
| Sauvage P1.2 | 80 | 80 | 100 |
| Ectopique 19.1 | 80 | 80 | 100 |
| Ectopique 20.1 | 80 | 80 | ND |

| | | | |
|---|---|---|---|
| ND : non déterminé | | | |

La formation des appressoria est aussi observée dans une condition artificielle où les spores sont mises à germer sur des membranes de téflon avec ou sans l'addition de méthionine (concentration de 1 mM dans nos conditions expérimentales). Ces membranes très hydrophobes miment la surface de la feuille ce qui permet d'induire la formation des appressorias et de mesurer facilement le taux de différentiation appressorial.

En conclusion, les mutants *met6*Δ::*hph* de *M. grisea* sont donc incapables de pénétrer dans la plante bien qu'ils différentient des appressoria. Ces résultats montrent que ces mutants ont des appressoria non-fonctionnels.

Des pots contenant des plants d'orge âgés de 3 semaines (et correspondant à l'émergence de la seconde feuille) sont soumis à une pulvérisation d'une suspension spores de *M. grisea* (10 ml d'eau contenant des spores à la concentration de 3.10⁴ spores/ml et 0.3% gélatine pour l'adhésion des gouttelettes d'eau sur les feuilles). Les observations des symptômes sont effectuées au moins pendant 8 jours après la pulvérisation. Les plantes d'orges traitées avec la souche sauvage (P1.2) et les transformants ectopiques (19.1 et 20.1) présentent des lésions nécrotiques provoquées par le développement du champignon dans les feuilles infectées. Par contre, aucun symptôme de maladie n'a été observé dans le cas de l'inoculation des mutants *met6*Δ::*hph* de *M. grisea*. Ces mutants sont donc considérés comme non pathogènes. De plus, cette étude indique, comme l'analyse réalisée avec les feuilles d'orge en survie, que le taux en méthionine (ou tout autres composés dérivant de la méthionine comme le SAM et le SMM) au sein des tissus de la feuille est insuffisant pour complémenter la carence dans la synthèse de la méthionine des mutants *met6*Δ::*hph*. Ainsi, la voie mise en évidence lors des expériences *in vitro* (complémentation en présence de SAM et de SMM) ne semble pas fonctionnelle chez le champignon lors de sa croissance dans la plante.

Les mutants *met6*Δ::*hph* obtenus, correspondent à la délétion de la phase codante du gène *MET6* qui est remplacée par le gène *hph* conférant la résistance à l'hygromycine. Ces mutants sont incapables de synthétiser la méthionine à partir de l'homocystéine et sont donc incapables de se multiplier sur un milieu minimum. L'addition de méthionine à ce milieu minimum est indispensable pour le développement de ces mutants. Les mutants *met6*Δ::*hph* ne provoquent aucun symptôme de maladie lors de leur inoculation à des feuilles d'orge soit en survie, soit de plantes entières, même à de fortes concentrations en spores. Ainsi, la synthèse de méthionine par la méthionine synthase est indispensable au développement du champignon aussi bien *in vitro* qu'*in planta.*

Les mutants *met6*Δ::*hph* peuvent différentier un appressorium en absence de méthionine. Cet aspect indique que la spore peut posséder une réserve non négligeable en méthionine permettant une synthèse des protéines et des métabolites nécessaires au développement de cette cellule. Cette réserve doit provenir de la méthionine qui a été fournie au mutant pour permettre sa croissance et sa sporulation sur le milieu à base de farine de riz utilisé (contenant 1 mM de méthionine). Par contre, l'absence de pénétration des mutants *met6*Δ::*hph* dans les feuilles indique que ceux-ci différentient des appressoria non-fonctionnels incapables de diriger la pénétration du champignon dans la plante. Il est probable que les appressoria mutants aient épuisé rapidement leurs réserves en méthionine. En effet, l'addition de méthionine aux spores mutantes permet une pénétration dans la plante et un début de développement dans la feuille. Cependant, celui-ci n'est pas complet et l'absence de formation de lésion sporulant suggère qu'une fois que la réserve en méthionine apportée aux spores est épuisée la plante est incapable de couvrir les besoins en méthionine des mutants.

### Exemple 6 : Méthodes pour le dosage et la caractérisation de molécules qui inhibent l'activité enzymatique de la méthionine synthase

La méthode implique la caractérisation de toutes molécules dont l'action inhibe la consommation des substrats ou la formation des produits déterminés selon des techniques directes ou indirectes (qui incluent l'utilisation d'une enzyme dite de couplage pour la mesure de l'activité de la méthionine synthase). La méthionine synthase catalyse une réaction irréversible en présence de l'homocystéine, de méthyltetrahydrofolate (n=1) ou ses dérivés polyglutamate (n≥3) en présence de différents cofacteurs (phosphate, magnésium, zinc) selon les dosages connus et décrits dans la littérature ^{12 13 14 15, 27}. La méthodologie inclue la détermination de la méthionine, des dérivés du folate produits par les techniques de séparation des composés par chromatographie en phase réverse par HPLC¹⁶. Le dosage du tétrahydrofolate produit au cours de la réaction après sa transformation en méthenyltétrahydrofolate peut être effectué au spectrophotomètre à 350 nm, puisque le substrat méthyltétrahydrofolate n'est pas détectable dans les conditions expérimentales décrites dans la procédure¹⁷. Une alternative proposée est le dosage de l'activité de la méthionine synthase en présence de la S-adénosylméthionine synthétase. Dans cet essai, la S-adénosylméthionine synthétase (AdoMetS) de *M. grisea* sera utilisée par préférence, mais toutes les AdoMetS peuvent être utilisées comme une enzyme dite de couplage. L'enzyme AdoMetS catalyse la réaction irréversible qui en présence de la méthionine, d'ATP et de magnésium produit du S-adénosylméthionine (SAM), du phosphate et du pyrophosphate. L'activité de la méthionine synthase est dosée en finale par la quantité de SAM, de phosphate ou du pyrosphophate produit, par une méthode colorimétrique et/ou au spectrophotomètre après transformation des produits en présence d'une enzyme de couplage ou par toute autre réaction chimique ou enzymatique permettant de mesurer l'activité de la méthionine synthase. Ces différentes méthodologies font l'objet de nombreuses descriptions dans la littérature et peuvent être adaptées selon l'expérimentateur^{18 19 20 21 22 23}. Par exemple, la sensibilité de la méthode de dosage de l'activité méthionine synthase en présence de la SAM synthétase peut être améliorée par l'addition d'une pyrophosphatase qui transforme le pyrophosphate en 2 moles de phosphate. Ainsi pour chaque mole de méthionine synthétisée, la méthode produit 3 moles de phosphate.

### Purification de la méthionine synthase de Magnaporthe grisea.

La production de la méthionine synthase en grande quantité est réalisée en utilisant les techniques utilisant des vecteurs d'expression pour la surproduction de la protéine dans les bactéries ou la levure. La technique utilise de préférence le clonage du cDNA dans un vecteur d'expression qui permet d'intégrer une extension His-Tag à l'extrémité N-terminale ou C-terminale de la protéine. Par exemple, dans le choix du vecteur pET-28b(+) (Novagen)²⁴, le cDNA de 2301 bp est cloné en *NdE*I et *EcoR*I selon les techniques traditionnelles de biologie moléculaire. La construction obtenue, appelé pET-28-Mg*MET*6, est introduite dans la souche bactérienne *Escherichia coli* BL21 de type DE3(pLysS) et l'expression est réalisée après induction par l'IPTG (0.5 mM). La culture des bactéries recombinantes est réalisée à 28°C pendant 4 heures. Les cellules sont ensuite récoltées par centrifugation et le culot obtenu est remis en suspension dans le tampon de lyse approprié pour la stabilité de la protéine. Après sonication de la préparation, la fraction soluble contenant la protéine recombinante obtenue après centrifugation, est déposée sur une colonne de type Ni-NTA agarose. La purification et l'élution de l'enzyme sont ensuite réalisées après plusieurs lavages successifs de la matrice avec une solution d'imidazole. La procédure suit le protocole défini par Qiagen²⁵.

Après élution, la fraction protéique contenant la méthionine synthase recombinante est concentrée par ultrafiltration et soumise à une filtration moléculaire sur PD10 (Pharmacia)²⁷ afin d'éliminer toute trace d'imidazole. La purification de la protéine recombinante peut être accompagnée d'une seconde étape consistant en une filtration moléculaire par chromatographie sur Superdex S200 (Pharmacia)²⁶ ou en une chromatographie d'échanges d'ions sur MonoQ HR10/10 (Pharmacia)²⁶. L'activité de la méthionine synthase est suivie au cours de la purification en utilisant le test de mesure directe approprié.

### Bibliographie

¹ Saint-Girons I, Parsot C, Zakin MM, Barzu O and Cohen GN (1988) Methionine biosynthesis in enterobacteria: biochemical, regulatory, and evolutionary aspects. Crit. Rev. Biochem. 23, S1-S42
² Droux M (2004) Sulfur assimilation and the role of sulfur in plant metabolism: a survey. Photosyn. Res. 79, 331-348
³ Thomas D and Surdin-Kerjan Y (1997) Metabolism of sulfur amino acids in Saccharomyces cerevisae. Microbiol. Mol. Biol. Rev. 61,503-532
⁴ Marzluf GA (1997) Molecular genetics of sulfur assimilation in filamentous fungi and yeast. Annu. Rev. Microbiol. 51, 73-96
⁵ Perkins DD, Radford A, Newmeyer D and Björkman M (1982) Chromosomal loci of Neurospora crassa. Microbiol. Rev. 46, 426-570
⁶ Paszewski A and Grabski J (1974) Regulation of S-amino acids biosynthesis in Aspergillus nidulans. Mol. Gen. Genet. 132, 307-320
⁷ Grynberg M, PiotrowskaM, Pizzinini E, Turner G and Paszewski A (2001) The Aspergillus nidulans metE gene is regulated by a second system independent from sulphur metabolite repression. Biochem. Biophys. Acta 1519, 78-84
⁸ Marchler-Bauer A, Anderson JB, DeWeese-Scott C, Fedorova ND, Geer LY, He S, Hurwitz DI, Jackson JD, Jacobs AR, Lanczycki CJ, Liebert CA, Liu C, Madej T, Marchler GH, Mazumder R, Nikolskaya AN, Panchenko AR, Rao BS, Shoemaker BA, Simonyan V, Song JS, Thiessen PA, Vasudevan S; Wang Y, Yamashita RA, Yin JJ and Bryant SH (2003) CDD: a curated Entrez database of conserved domain alignments. Nucleic Acids Res. 31, 383-387.
⁹ Ferrer JL, Ravanel S, Robert M and Dumas R (2004) Crystal structures of cobalamin-independent methionine synthase complexed with Zn, homocysteine and methyltetrahydrofolate. J. Biol. Chem., in press.
¹⁰ Latijnhouwers M, de Wit PJ and Govers F (2003) Oomycetes and fungi: similar weaponry to attack plants. Trends Microbiol. 11, 462-469
¹¹ Thomas D, Becker A and Surdin-Kerjan Y (2000) Reverse methionine biosynthesis from S-adenosylmethionine in Eukaryotic cells. J. Biol. Chem. 275, 40718-40724
¹² Eichel J, Gonzalez JC, Hotze M, Matthews RG and Schröder J (1995) Vitamin B12-indépendent L-methionine synthase from a higher plant (Cataranthus roseus): molecular characterization, regulation, heterologous expression, and enzyme properties. Eur. J. Biochem. 230, 1053-1058
¹³ Eckermann C, Eichel J and Schroder J (2000) Plant methionine synthase: new insights into properties and expression. Biol. Chem. 381, 695-703
¹⁴ Gonzalez JC, Peariso K, Penner-Hahn JE and Matthews RG (1996) Cobalamin-independent methionine synthase from Escherichia coli: involvement: a zinc mettalloenzyme. Biochemistry 35, 12228-12234
¹⁵Whifield CD, Steers EJ Jr and Weissbach H (1970) Purification and properties of 5-methyltetrahydropteroyltriglutamate-homocysteine transmethylase. J. Biol. Chem. 245, 390-401.
¹⁶ Huang L, Zhang J, Hayakawa T and Tsuge H (2001) Assays of methylenetetrahydrofolate reductase and methionine synthase activities by monitoring 5-methyltetrahydrofolate and tetrahydrofolate using High-Performance Liquid Chromatography Liquid Chromatography with fluorescence detection. Anal. Biochem. 299, 253-259
¹⁷ Drummond JT, Jarrett J, Gonzalez JC, Huang S and Matthews RG (1995) Characterization of nonradioactive assays for cobalamin- dependent and cobalamin-independent methionine synthase enzymes. Anal. Biochem. 228, 323-329
¹⁸ Lanzetta PA, Alvarez LJ, Reinach PS and Candia OA (1979) An improved assay for nanomole amounts of inorganic phosphate. Anal. Biochem. 100, 95-97
¹⁹ Rosalyn HU, Haugland RP, Malekzadeh MN and Haugland RP (1996) A spectrophotometric method to measure enzymatic activity in reactions that generate inorganic pyrophosphate. Anal. Biochem. 243, 41-45
²⁰ Cannon LM, Butler FN, Wan W and Zhou ZS (2002) A stereospecific colorimetric assay for (S,S)-adenosylmethionine quantification based on thiopurine methyltransferase-catalyzed thiol methylation. Anal. Biochem. 308, 358-363
²¹ Katewa SD and Katyare SS (2003) A simplified method for inorganic phosphate determination and its application for phosphate analysis in enzyme assays. Anal. Biochem. 323, 180-187
²² Vazquez MJ, Rodriguez B, Zapatero C and Tew DG (2003) Determination of phosphate in nanomolar range by an enzyme-coupling fluorescent method. Anal. Biochem. 320, 292-298
²³ Gawronski JD and Benson DR (2004) Microtiter assay for glutamine synthetase biosynthetic activity using inorganic phosphate detection. Anal. Biochem. 327, 114-118
²⁴ Novagen : http://www.merckbiosciences.co.uk/g.asp?f=NVG/hone.html
²⁵ Qiagen : http://www1.giagen.com
²⁶ Pharmacia : https://chromatography.amershambiosciences.com
²⁷ Ravanel S., Block MA., Rippert P., Jabrin S., Curien G., Rébeillé F et Douce R. (2004) Methionine metabolism in plants : chloroplasts are autonomous for de novo methionine synthesis and can import S-adenosylmethionine from the cytosol. J. Biol. Chem. 279, 22548-22557.
²⁸ Pascon R.C., Ganous T.M., Kingbury J.M., Cox G.M. ans McCusker J.H. (2004) Cryptococcus neoformans methionine synthase : expression analysis and requirement for vehicule.

## Revendications

1. Procédé pour l'identification de composés antifongiques pour le traitement des maladies fongiques des espèces végétales cultivées comprenant l'identification de composés inhibant l'activité enzymatique de la méthionine synthase d'un champignon phytopathogène.

2. Procédé selon la revendication 1, **caractérisé en ce que** la méthionine synthase est issue de *Magnaporthe grisea*.

3. Procédé selon la revendication 2, **caractérisé en ce que** la méthionine synthase comprend la SEQ ID NO: 3.

4. Procédé selon la revendication 2, **caractérisé en ce que** la méthionine synthase est codée par une séquence comprenant la SEQ ID NO: 1 ou la SEQ ID NO: 2.

5. Procédé selon la revendication 1, **caractérisé en ce que** la méthionine synthase est issue de *Ustilago maydis*.

6. Procédé selon la revendication 5, **caractérisé en ce que** la méthionine synthase comprend la SEQ ID NO: 18.

7. Procédé selon la revendication 5, **caractérisé en ce que** la méthionine synthase est codée par une séquence comprenant la SEQ ID NO: 16 ou la SEQ ID NO: 17

8. Procédé selon la revendication 1, **caractérisé en ce que** la méthionine synthase est issue de *Phytophtora infestans*.

9. Procédé selon la revendication 8, **caractérisé en ce que** la méthionine synthase comprend la SEQ ID NO: 20.

10. Procédé selon la revendication 9, **caractérisé en ce que** la méthionine synthase est codée par une séquence comprenant la SEQ ID NO: 19.

11. Procédé selon l'une des revendications 1 à 10, **caractérisé en ce que** l'identification de composés inhibant l'activité enzymatique de la méthionine synthase comprend les étapes suivantes:
- mettre en contact ledit composé avec la méthionine synthase en présence d'homocystéine et de méthyltétraglutamate ou de ses dérivés polyglutamate et de cofacteurs; et
- mesurer ladite activité enzymatique.

12. Procédé selon la revendication 11, **caractérisé en ce que** l'identification de composés inhibant l'activité enzymatique de la méthionine synthase comprend les étapes suivantes:
- mettre en contact ledit composé avec la méthionine synthase en présence d'homocystéine et de méthyltétraglutamate ou de ses dérivés polyglutamate et de cofacteurs, et
- mesurer la formation de méthionine.

13. Procédé selon la revendication 12, **caractérisé en ce que** l'identification de composés inhibant l'activité enzymatique de la méthionine synthase comprend les étapes suivantes:
- mettre en contact ledit composé avec la méthionine synthase en présence d'homocystéine, de méthyltétrahydrofolate et de phosphate, magnésium et zinc, et
- mesurer la formation de méthionine.

14. Procédé selon l'une des revendications 1 à 10, **caractérisé en ce que** l'identification de composés inhibant l'activité enzymatique de la méthionine synthase comprend les étapes suivantes:
- mettre en contact ledit composé avec la méthionine synthase en présence d'homocystéine, de méthyltétrahydrofolate ou de ses dérivés polyglutamate, de S-adenosylmethionine synthétase, d'ATP et de Mg et de cofacteurs, et
- mesurer la formation de S-adénosylméthionine, phosphate ou de pyrophosphate.

15. Procédé selon la revendication 14, **caractérisé en ce que** l'identification de composés inhibant l'activité enzymatique de la méthionine synthase comprend les étapes suivantes:
- mettre en contact ledit composé avec la méthionine synthase en présence d'homocystéine, de méthyltétrahydrofolate ou de ses dérivés polyglutamate, de S-adenosylmethionine synthétase, d'ATP et de Mg et de cofacteurs, et
- mesurer la formation de phosphate.

16. Procédé selon l'une des revendications 1 à 10, **caractérisé en ce que** l'identification de composés inhibant l'activité enzymatique de la méthionine synthase comprend les étapes suivantes:
- exprimer la méthionine synthase dans un organisme hôte;
- purifier la méthionine synthase produite par ledit organisme hôte;
- mettre en contact ledit composé avec ladite méthionine synthase purifiée en présence d'homocystéine et de méthyltétrahydrofolate ou de ses dérivés polyglutamate et de cofacteurs; et
- mesurer l'activité enzymatique.

17. Procédé selon la revendication 16, **caractérisé en ce que** l'identification de composés inhibant l'activité enzymatique de la méthionine synthase comprend les étapes suivantes:
- exprimer la méthionine synthase dans un organisme hôte;
- purifier la méthionine synthase produite par ledit organisme hôte;
- mettre en contact ledit composé avec ladite méthionine synthase purifiée en présence d'homocystéine, de méthyltétrahydrofolate et de phosphate, magnésium et zinc et
- mesurer la formation de méthionine

18. Procédé selon l'une des revendications 1 à10, **caractérisé en ce que** l'identification de composés inhibant l'activité enzymatique de la méthionine synthase comprend les étapes suivantes:
- exprimer la méthionine synthase dans un organisme hôte;
- purifier la méthionine synthase produite par ledit organisme hôte;
- mettre en contact ledit composé avec ladite méthionine synthase purifiée en présence d'homocystéine, de méthyltétrahydrofolate ou de ses dérivés polyglutamate, de S-adenosylmethionine synthétase, d'ATP et de Mg et de cofacteurs, et
- mesurer la formation de S-adénosylméthionine, de phosphate ou de pyrophosphate.

19. Procédé selon l'une des revendications 1 à 18, comprenant une étape supplémentaire dans laquelle on détermine si lesdits composés inhibant l'activité enzymatique de la méthionine synthase inhibent la croissance et/ou la pathogénie des champignons.

## Claims

1. Method for identifying antifungal compounds for the treatment of fungal diseases of crop plant species, comprising the identification of compounds which inhibit the enzymatic activity of methionine synthase of a phytopathogenic fungus.

2. Method according to Claim 1, **characterized in that** the methionine synthase is derived from *Magnaporthe grisea*.

3. Method according to Claim 2, **characterized in that** the methionine synthase comprises SEQ ID NO: 3.

4. Method according to Claim 2, **characterized in that** the methionine synthase is encoded by a sequence comprising SEQ ID NO: 1 or SEQ ID NO: 2.

5. Method according to Claim 1, **characterized in that** the methionine synthase is derived from *Ustilago maydis*.

6. Method according to Claim 5, **characterized in that** the methionine synthase comprises SEQ ID NO: 18.

7. Method according to Claim 5, **characterized in that** the methionine synthase is encoded by a sequence comprising SEQ ID NO: 16 or SEQ ID NO: 17.

8. Method according to Claim 1, **characterized in that** the methionine synthase is derived from *Phytophthora infestans*.

9. Method according to Claim 8, **characterized in that** the methionine synthase comprises SEQ ID NO: 20.

10. Method according to Claim 9, **characterized in that** the methionine synthase is encoded by a sequence comprising SEQ ID NO: 19.

11. Method according to one of Claims 1 to 10, **characterized in that** the identification of compounds which inhibit the enzymatic activity of methionine synthase comprises the following steps:
- bringing said compound into contact with methionine synthase in the presence of homocysteine and of methyl tetraglutamate or its polyglutamate derivatives, and of cofactors; and
- measuring said enzymatic activity.

12. Method according to Claim 11, **characterized in that** the identification of compounds which inhibit the enzymatic activity of methionine synthase comprises the following steps:
- bringing said compound into contact with methionine synthase in the presence of homocysteine and of methyltetraglutamate or its polyglutamate derivatives, and of cofactors, and
- measuring the formation of methionine.

13. Method according to Claim 12, **characterized in that** the identification of compounds which inhibit the enzymatic activity of methionine synthase comprises the following steps:
- bringing said compound into contact with methionine synthase in the presence of homocysteine, of methyl tetrahydrofolate and of phosphate, magnesium and zinc, and
- measuring the formation of methionine.

14. Method according to one of Claims 1 to 10, **characterized in that** the identification of compounds which inhibit the enzymatic activity of methionine synthase comprises the following steps:
- bringing said compound into contact with methionine synthase in the presence of homocysteine, of methyl tetrahydrofolate or its polyglutamate derivatives, of S-adenosylmethionine synthetase, of ATP and of Mg, and of cofactors, and
- measuring the formation of S-adenosylmethionine, phosphate or pyrophosphate.

15. Method according to Claim 14, **characterized in that** the identification of compounds which inhibit the enzymatic activity of methionine synthase comprises the following steps:
- bringing said compound into contact with methionine synthase in the presence of homocysteine, of methyl tetrahydrofolate or its polyglutamate derivatives, of S-adenosylmethionine synthetase, of ATP and of Mg, and of cofactors, and
- measuring the formation of phosphate.

16. Method according to one of Claims 1 to 10, **characterized in that** the identification of compounds which inhibit the enzymatic activity of methionine synthase comprises the following steps:
- expressing methionine synthase in a host organism;
- purifying the methionine synthase produced by said host organism;
- bringing said compound into contact with said purified methionine synthase in the presence of homocysteine and of methyl tetrahydrofolate or its polyglutamate derivatives, and of cofactors; and
- measuring the enzymatic activity.

17. Method according to Claim 16, **characterized in that** the identification of compounds which inhibit the enzymatic activity of methionine synthase comprises the following steps:
- expressing methionine synthase in a host organism;
- purifying the methionine synthase produced by said host organism;
- bringing said compound into contact with said purified methionine synthase in the presence of homocysteine, of methyl tetrahydrofolate and of phosphate, magnesium and zinc, and
- measuring the formation of methionine.

18. Method according to one of Claims 1 to 10, **characterized in that** the identification of compounds which inhibit the enzymatic activity of methionine synthase comprises the following steps:
- expressing methionine synthase in a host organism;
- purifying the methionine synthase produced by said host organism;
- bringing said compound into contact with said purified methionine synthase in the presence of homocysteine, of methyl tetrahydrofolate or its polyglutamate derivatives, of S-adenosylmethionine synthetase, of ATP and of Mg, and of cofactors, and
- measuring the formation of S-adenosylmethionine, of phosphate or of pyrophosphate.

19. Method according to one of Claims 1 to 18, comprising an additional step in which it is determined whether said compounds which inhibit the enzymatic activity of methionine synthase inhibit fungal growth and/or pathogenesis.

## Patentansprüche

1. Verfahren zum Identifizieren von antifungalen Verbindungen für die Behandlung von Pilzkrankheiten bei Nutzpflanzen, bei den Verbindungen, die die Enzymaktivität der Methioninsynthase eines phytopathogenen Pilzes hemmen, identifiziert werden.

2. Verfahren nach Anspruch 1, **dadurch gekennzeichnet, daß** die Methioninsynthase aus *Magnaporthe grisea* stammt.

3. Verfahren nach Anspruch 2, **dadurch gekennzeichnet, daß** die Methioninsynthase SEQ ID NO: 3 umfaßt.

4. Verfahren nach Anspruch 2, **dadurch gekennzeichnet, daß** die Methioninsynthase von einer Sequenz umfassend SEQ ID NO: 1 oder SEQ ID NO: 2 codiert wird.

5. Verfahren nach Anspruch 1, **dadurch gekennzeichnet, daß** die Methioninsynthase aus *Ustilago maydis* stammt.

6. Verfahren nach Anspruch 5, **dadurch gekennzeichnet, daß** die Methioninsynthase SEQ ID NO: 18 umfaßt.

7. Verfahren nach Anspruch 5, **dadurch gekennzeichnet, daß** die Methioninsynthase von einer Sequenz umfassend SEQ ID NO: 16 oder SEQ ID NO: 17 codiert wird.

8. Verfahren nach Anspruch 1, **dadurch gekennzeichnet, daß** die Methioninsynthase aus *Phytophthora infestans* stammt.

9. Verfahren nach Anspruch 8, **dadurch gekennzeichnet, daß** die Methioninsynthase SEQ ID NO: 20 umfaßt.

10. Verfahren nach Anspruch 9, **dadurch gekennzeichnet, daß** die Methioninsynthase von einer Sequenz umfassend SEQ ID NO: 19 codiert wird.

11. Verfahren nach einem der Ansprüche 1 bis 10, **dadurch gekennzeichnet, daß** die Identifikation von Verbindungen, die die Enzymaktivität der Methioninsynthase hemmen, die folgenden Schritte umfaßt:
- Inkontaktbringen der Verbindung mit der Methioninsynthase in Gegenwart von Homocystein und Methyltetraglutamat oder seinen Polyglutamatderivaten und Cofaktoren; und
- Bestimmen der Enzymaktivität.

12. Verfahren nach Anspruch 11, **dadurch gekennzeichnet, daß** die Identifikation von Verbindungen, die die Enzymaktivität der Methioninsynthase hemmen, die folgenden Schritte umfaßt:
- Inkontaktbringen der Verbindung mit der Methioninsynthase in Gegenwart von Homocystein und Methyltetraglutamat oder seinen Polyglutamatderivaten und Cofaktoren; und
- Bestimmen der Methioninbildung.

13. Verfahren nach Anspruch 12, **dadurch gekennzeichnet, daß** die Identifikation von Verbindungen, die die Enzymaktivität der Methioninsynthase hemmen, die folgenden Schritte umfaßt:
- Inkontaktbringen der Verbindung mit der Methioninsynthase in Gegenwart von Homocystein und Methyltetrahydrofolat sowie von Phosphat, Magnesium und Zink; und
- Bestimmen der Methioninbildung.

14. Verfahren nach einem der Ansprüche 1 bis 10, **dadurch gekennzeichnet, daß** die Identifikation von Verbindungen, die die Enzymaktivität der Methioninsynthase hemmen, die folgenden Schritte umfaßt:
- Inkontaktbringen der Verbindung mit der Methioninsynthase in Gegenwart von Homocystein und Methyltetrahydrofolat oder seinen Polyglutamatderivaten, von S-Adenosylmethioninsynthetase, von ATP und von Mg und Cofaktoren, und
- Bestimmen der S-Adenosylmethionin-, Phosphat-oder Pyrophosphatbildung.

15. Verfahren nach Anspruch 14, **dadurch gekennzeichnet, daß** die Identifikation von Verbindungen, die die Enzymaktivität der Methioninsynthase hemmen, die folgenden Schritte umfaßt:
- Inkontaktbringen der Verbindung mit der Methioninsynthase in Gegenwart von Homocystein und Methyltetrahydrofolat oder seinen Polyglutamatderivaten, von S-Adenosylmethioninsynthetase, von ATP und von Mg und Cofaktoren, und
- Bestimmen der Phosphatbildung.

16. Verfahren nach einem der Ansprüche 1 bis 10, **dadurch gekennzeichnet, daß** die Identifikation von Verbindungen, die die Enzymaktivität der Methioninsynthase hemmen, die folgenden Schritte umfaßt:
- Exprimieren der Methioninsynthase in einem Wirtsorganismus;
- Aufreinigen der von diesem Wirtsorganismus produzierten Methioninsynthase;
- Inkontaktbringen der Verbindung mit der aufgereinigten Methioninsynthase in Gegenwart von Homocystein und Methyltetrahydrofolat oder seinen Polyglutamatderivaten und Cofaktoren; und
- Bestimmen der Enzymaktivität.

17. Verfahren nach Anspruch 16, **dadurch gekennzeichnet, daß** die Identifikation von Verbindungen, die die Enzymaktivität der Methioninsynthase hemmen, die folgenden Schritte umfaßt:
- Exprimieren der Methioninsynthase in einem Wirtsorganismus;
- Aufreinigen der von diesem Wirtsorganismus produzierten Methioninsynthase;
- Inkontaktbringen der Verbindung mit der aufgereinigten Methioninsynthase in Gegenwart von Homocystein und Methyltetrahydrofolat oder Phosphat, Magnesium und Zink; und
- Bestimmen der Methioninbildung.

18. Verfahren nach einem der Ansprüche 1 bis 10, **dadurch gekennzeichnet, daß** die Identifikation von Verbindungen, die die Enzymaktivität der Methioninsynthase hemmen, die folgenden Schritte umfaßt:
- Exprimieren der Methioninsynthase in einem Wirtsorganismus;
- Aufreinigen der von diesem Wirtsorganismus produzierten Methioninsynthase;
- Inkontaktbringen der Verbindung mit der aufgereinigten Methioninsynthase in Gegenwart von Homocystein und Methyltetrahydrofolat oder seinen Polyglutamatderivaten, von S-Adenosylmethioninsynthetase, von ATP und Mg und Cofaktoren, und
- Bestimmen der S-Adenosylmethionin-, Phosphat-oder Pyrophosphatbildung.

19. Verfahren nach einem der Ansprüche 1 bis 18, das einen zusätzlichen Schritt umfaßt, in dem man bestimmt, ob die Verbindungen, die die Enzymaktivität der Methioninsynthase hemmen, das Wachstum und/oder die Pathogenität von Pilzen hemmen.
